(19) Europäisches Patentamt
European Patent Office
Office européen des brevets

(11) Publication number: **0 254 545 B1**

(12) **EUROPEAN PATENT SPECIFICATION**

(45) Date of publication of patent specification: **06.10.93**

(51) Int. Cl.5: **C07D 295/12**, C07D 215/12, C07D 213/61, C07D 263/56, C07D 209/48, C07D 333/60, C07D 307/79, C07C 235/00, C07D 521/00

(21) Application number: **87306463.8**

(22) Date of filing: **22.07.87**

The file contains technical information submitted after the application was filed and not included in this specification

# (54) Diamine compounds.

(30) Priority: **25.07.86 GB 8618188**

(43) Date of publication of application:
**27.01.88 Bulletin 88/04**

(45) Publication of the grant of the patent:
**06.10.93 Bulletin 93/40**

(84) Designated Contracting States:
**BE CH DE ES FR GB IT LI NL**

(56) References cited:
**EP-A- 0 126 612**
**DE-B- 1 175 247**
**FR-A- 1 593 024**
**EP-A- 0 261 842**
**FR-A- 1 455 890**
**GB-A- 2 136 801**

(73) Proprietor: **IMPERIAL CHEMICAL INDUSTRIES PLC**
**Imperial Chemical House,**
**Millbank**
**London SW1P 3JF(GB)**

(72) Inventor: **Costello, Gerard Francis**
**16 Millbank Drive**
**Macclesfield Cheshire SK10 3DR(GB)**

(74) Representative: **Mack, John Richard et al**
**ICI Group Patents Services Dept.**
**PO Box 6**
**Shire Park**
**Bessemer Road**
**Welwyn Garden City Herts, AL7 1HD (GB)**

EP 0 254 545 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to 1,2-ethylene diamine compounds and the salts thereof, processes for their preparation and pharmaceutical compositions containing the said compounds and the pharmaceutically acceptable salts thereof. The compounds and their pharmaceutically acceptable salts possess analgesic activity.

US Patent specification No. 4,145,435 discloses cis and trans-N-(2-aminocycloaliphatic)-2-arylacetamide derivatives as possessing potent analgesic activity. European Patent Publication No. 110 869 discloses trans-N-(2-aminocyclohexyl)-2-thienylacetamide derivatives and European Patent Publication No. 126,612 discloses cis- and trans-N-[2-(2,5-dihydro-1H-pyrrol-1-yl)cycloaliphatic]-2-benzeneacetamide derivatives all possessing analgesic activity.

The present invention is based on the discovery that the cycloaliphatic ring present in the above-mentioned compounds is not essential for activity, and further on the discovery that provided the stereochemical centre is maintained at the carbon adjacent to the amidic nitrogen atom, the open chain ethylene diamine structure of the compounds of the present invention enables a wide range of substituents to be introduced which would not otherwise have been possible, whereby compounds having a significantly improved analgesic potency over corresponding known compounds are obtained.

According to one feature of the present invention there are provided compounds of formula I (as set out hereinafter) wherein $R^1$ represents a $C_{6-10}$ aryl group optionally substituted by one, two or three substituents independently selected from halogen, hydroxy, trifluoromethyl, cyano, nitro, amino, aminocarbonyl, carboxy, sulphonic acid, ($C_{1-6}$ alkoxy)carbonyl, $C_{1-6}$ alkyl sulphide, $C_{1-6}$ alkyl sulphoxide, $C_{1-6}$ alkyl sulphone, $C_{1-6}$ alkanoyl, $C_{1-6}$ alkoxy, $C_{3-6}$ alkenyloxy, $C_{3-6}$ alkynyloxy, $C_{1-6}$ acylamino, $C_{1-6}$ acylmethylamino, $C_{1-6}$ alkyl, $C_{1-6}$ monoalkylamino, ($C_{1-6}$ monoalkylamino)carbonyl, and a group of formula II (as set out hereinafter in which A is -CO- or a single bond and $R^6$ and $R^7$ which may be the same or different each represent a $C_{1-6}$ alkyl group or $R^6$ and $R^7$ together with the intervening nitrogen atom represents a cyclic amine with 4 to 7 ring atoms and where appropriate the oxides thereof), or $R^1$ represents a heterocyclic moiety comprising a 5-or 6- membered heterocyclic ring containing one or two heteroatoms independently selected from oxygen, nitrogen and sulphur, the ring optionally being fused with a benzene ring and the heterocyclic and/or benzene ring being optionally substituted on carbon by one or more substituents selected from amino, halogen, hydroxy (and ketotautomers thereof) $C_{1-6}$ alkyl, $C_{1-6}$ alkoxy, $C_{3-6}$ alkenyloxy and $C_{3-6}$ alkynyloxy, any nitrogen heteroatom optionally carrying an oxygen atom or a hydroxy or $C_{1-3}$ alkyl group;

X represents a single bond, $-CH_2-$, $-OCH_2-$, $-SCH_2-$, $-S(O)-CH_2-$, $-S(O)_2-CH_2-$ or $-CH_2-CH_2-$;

$R^2$ represents hydrogen or $C_{1-3}$ alkyl;

$R^3$ represents an alkyl, cycloalkyl or cycloalkylmethyl group having up to 7 carbon atoms, the cycloalkyl moiety where present, having 3 to 6 carbon atoms, said group optionally being substituted by one or more substituents selected from hydroxy, amino, amidino, guanidino, aminocarbonyl, carboxy, $C_{1-6}$ alkoxy, ($C_{1-6}$ alkoxy)carbonyl, ($C_{3-6}$ alkenyloxy)carbonyl, ($C_{3-6}$ alkynyloxy)carbonyl, $C_{1-6}$ alkanoyloxy, $C_{1-6}$ alkylsulphide, $C_{1-6}$ alkylsulphoxide, $C_{1-6}$ alkylsulphone, $C_{1-6}$ (monoalkylamino)carbonyl, $C_{1-6}$ acylamino, $C_{1-6}$ acylmethylamino, $C_{1-6}$ monoalkylamino, a group of formula II (as herein defined);

or $R^3$ represents the group $-B-R^1_a$ in which B represents $-CH_2-$, $-CH(CH_3)-$ or a single bond and $R^1_a$ represents an optionaally substituted $C_{6-10}$ carbocyclic aryl group as defined for $R^1$,

or $R^3$ represents the group $-D-R_b$ in which D represents a single bond, $-CH_2-$, $-CH(CH_3)-$, $-CH_2-O-$, $-CH(CH_3)-O-$, $-CH_2-S-$, $-CH(CH_3)-S-$, $-CH_2-NH-$ or $-CH(CH_3)-NH-$ and $R_b$ represents a 4-to 6-membered heterocyclic ring containing up to 4 heteroatoms selected from oxygen, sulphur and nitrogen, the heterocyclic ring optionally being substituted on nitrogen or sulphur by oxygen or on nitrogen by hydroxy or $C_{1-3}$ alkyl and/or the ring optionally being substituted on carbon by one or more substituents selected from amino, hydroxy, thio (and their tautomers), cyano, halogen, $C_{1-3}$ alkoxy, $C_{1-3}$ monoalkylamino, $C_{1-3}$ acylamino, $C_{1-3}$ acylmethylamino, $C_{1-3}$ alkylthio and the group of formula II as herein defined;

and $R^4$ and $R^5$, which may be the same or different, each represents a $C_{3-5}$ alkenyl, $C_{3-5}$ alkynyl, $C_{1-6}$ alkyl, or $C_{4-7}$ cycloalkylalkyl group;

or $R^4$ and $R^5$ together with the intervening nitrogen atom represent a 4-7-membered heterocyclic ring which optionally contains a further heteroatom selected from oxygen and sulphur] or racemates thereof, and the salts of said compounds or racemates.

Unless otherwise specified, references herein to alkyl, alkenyl and alkynyl groups include such groups in both straight chain and branched forms.

The compounds of formula I possess an asymmetric carbon atom, that being the carbon atom carrying the substituent $R^3$. It will be understood that the present invention includes the racemates of the compounds

of formula I as well as the optically active enantiomer having the absolute configuration at substituted $R^3$ which would be obtained by synthesis from a natural (L)-alpha-amino acid as indicated in formula I which compounds possess the useful physiological properties of the compositions of the invention hereinafter described. Furthermore each of the substituents $R^1$ and $R^3$ may contain at least one asymmetric carbon atom and it will be understood that the present invention encompasses the racemic form of such compounds as well as any individual optical isomers thereof which possess the useful physiological properties of the compositions of the present invention as hereinafter defined, it being common general knowledge to those skilled in the art how such isomers may be separated and how their physiological properties may be determined.

The present invention encompasses the salts of the compounds of formula I or racemate thereof. It will be appreciated, however, that for pharmaceutical use, the salts referred to will be pharmaceutically acceptable, but other salts may find use, for example in the preparation of compounds of formula I and their pharmaceutically acceptable salts. The salts of the present invention include acid addition salts with for example mineral acids such as hydrochloric acid and organic acids such as maleic and fumaric acid.

The compounds of the present invention, in general, have a distribution coefficient between octanol and aqueous buffer of 1 or greater at pH 7.4. The distribution coefficient of a compound of the present invention may be determined as described hereinafter.

Where $R^1$, and/or $R^3$ represents a moiety which incorporates a group of formula II in which $R^6$ and $R^7$ together with the intervening nitrogen atom represent a cyclic amine with 4 to 7 ring atoms, particular values for the said cyclic amine are the azetidinyl, pyrrolidinyl, piperazinyl, piperidinyl, morpholinyl or 1,4-thiazinyl group or where appropriate an oxide thereof.

Where $R^3$ represents the group $-D-R_b$ and D represents $-CH_2-O-$, $-CH(CH_3)-O-$, $-CH_2-S-$, $-CH(CH_3)-S-$, $CH_2-NH-$ or $-CH(CH_3)-NH-$ it will be appreciated that the methylene group thereof is bonded to the asymmetric carbon atom of the compound of formula I whilst the heteroatom (O, S or N) is bonded to $R_b$.

Where $R^3$ represents the group $-D-R_b$, $R_b$ is preferably pyridine, pyrimidine, triazine, triazole, oxazole, morpholine, isoxazole, thiazole, pyrazine or pyridazine optionally substituted as hereinbefore defined for $R_b$.

Where $R^4$ and $R^5$ together with the intervening nitrogen atom represents a 4-7 membered ring optionally containing a further heteroatom, particular values for said ring are the pyrrolidinyl, pyrrolinyl, morpholinyl or piperidinyl group.

In the compounds of formula I, particular values for $R^1$ are a phenyl or naphthyl group optionally substituted by one, two or three substituents, particularly one or two substituents, selected from halogen (e.g. chlorine, bromine or fluorine), trifluoromethyl, cyano, nitro, amino, $C_{1-6}$ alkyl or $C_{1-6}$ alkoxy; further particular values for $R^1$ are a 5-or 6-membered heterocyclic ring containing one or two heteroatoms selected from oxygen, nitrogen or sulphur, the heterocyclic ring optionally being fused with a benzene ring, and the heterocyclic ring being optionally substituted by halogen and/or hydroxy and link to the remainder of the compound of formula I either directly or via the fused benzene ring. Further particular values for $R^1$ are a phenyl or naphthyl group substituted by one or two substituents selected from halogen, (e.g. chlorine, brominde or fluorine), trifluoromethyl, cyano, nitro, $C_{1-6}$alkyl or $C_{1-6}$ alkoxy, or $R^1$ is a 5- or 6-membered heterocyclic ring containing one or two heteroatoms selected from oxygen, nitrogen or sulphur, the heterocylic ring being fused with a benzene ring, and the heterocyclic and/or benzene ring being optionally substituted by halogen and/or hydroxy, linkage to the remainder of the compound of formula I being via the heterocyclic ring.

Particular values for X are $-CH_2$, $-OCH_2$, $-SCH_2-$ or $-CH_2-CH_2-$, especially $-CH_2-$.

Particular values for $R^2$ are hydrogen, methyl, ethyl or isopropyl, especially methyl.

Particular values for $R^3$ are an alkyl (in particular $C_{1-4}$ alkyl), cycloalkyl or cycloalkylalkyl (particularly alkyl) group having up to 7 carbon atoms in which the cycloalkyl moiety where present, has 3 to 6 carbon atoms, the group optionally being substituted by hydroxy, $C_{1-6}$ alkylsulphide, $C_{1-6}$ alkylsulphinyl, $C_{1-6}$ alkoxy and/or less preferably $C_{1-6}$ alkanoyloxy. Where $R^3$ represents an aliphatic moiety $R^3$ is preferably alkyl (advantageously $C_{1-4}$ alkyl) optionally substituted as indicated in the particular values above. Further particular values for $R^3$ are phenyl optionally substituted by one, two or three substituents, particularly one or two substituents, selected from hydroxy, nitro, $C_{1-6}$ alkylsulphide, $C_{1-6}$ alkysulphinyl, $C_{1-6}$ alkylsulphonyl, $C_{1-6}$ alkoxy, amino, $C_{1-6}$ alkylamino or a $C_{1-6}$ acylamino group. Further particular values for $R^3$ include groups of the formula $-D-R_b$ in which D represents a single bond or a $-CH(CH_3)-$ group, and $R_b$ represents a 5- or 6-membered heterocyclic ring containing one or two heteroatoms selected from oxygen, nitrogen or sulphur. A further particular value for $R^3$ is benzyl.

Particular values for $R^4$ and $R^5$, which may be the same or different, are each a $C_{1-6}$ alkyl group or a $C_{3-5}$ alkenyl group. Particular values for $R^4$ and $R^5$ together with the intervening nitrogen atom are 5- or 6-membered heterocyclic rings, especially where the nitrogen atom is the sole heteroatom.

More particular values for $R^1$ are, halophenyl, (e.g. chlorophenyl such as 2-chlorophenyl, 3-chlorophenyl or 4-chlorophenyl, dihalophenyl such as dichlorophenyl e.g. 3,4-dichlorophenyl, bromophenyl such as 4-bromophenyl, or fluorophenyl such as 3,4-difluorophenyl, 3-fluorophenyl or 4-fluorophenyl) trifluoromethylphenyl such as 4-(trifluoromethyl)phenyl, methoxyphenyl such as 4-methoxyphenyl, methylphenyl such as 4-methylphenyl, nitrophenyl such as 4-nitrophenyl, cyanophenyl such as 4-cyanophenyl, naphthyl such as 2-naphthyl, benzothienyl such as 3-benzothienyl, benzofuranyl such as 3-benzofuranyl, benzoxazolyl such as 2-benzoxazolyl; benzisoxazolyl optionally substituted by halogen, such as fluorine, for example 3-benzisoxazolyl, 3-(5-fluoro)benzisoxazolyl or 3-(6-fluoro)benzisoxazolyl; benzimidazolyl optionally substituted by halogen such as fluorine, for example 2-benzimidazolyl, 2-(5-fluoro)benzimidazolyl; 2-(1,3-dioxoisoindolinyl) and chloropyridyl such as 2-chloro-5-pyridyl.

A more particular value for $R^2$ is methyl.

More particular values for $R^3$ are methyl, ethyl, isopropyl, n-propyl, isobutyl, sec-butyl, t-butyl, methylthiomethyl, 1-methylthioethyl, 2-(methylthio)ethyl, 1-hydroxyethyl, 2-hydroxyethyl, $(C_{1-4})$-alkoxymethyl (for example methoxymethyl, ethoxymethyl, propoxymethyl and butoxymethyl, particularly t-butoxymethyl), $C_{1-4}$alkoxyethyl (for example 1-ethoxyethyl, 1-propoxyethyl and 1-butoxyethyl, particularly 1-t-butoxyethyl but especially 1-methoxyethyl), cyclohexylmethyl, 2-(methylsulphinyl)ethyl, phenyl, hydroxyphenyl such as 4-hydroxyphenyl, or 3-hydroxyphenyl, nitrophenyl such as 3-nitrophenyl, methylthiophenyl such as 4-methylthiophenyl, methylsulphonyl such as 4-(methylsulphonyl)phenyl, methylsulphinylphenyl such as 4-methylsulphinyl)phenyl, hydroxymethylphenyl such as 4-(hydroxymethylphenyl, methoxyphenyl such as 3-methoxyphenyl, 4-methoxyphenyl or 3,4-dimethoxyphenyl, propoxyphenyl such as 3-propoxyphenyl or 3-(1-propoxy)phenyl, aminophenyl such as 3-aminophenyl; methylaminophenyl such as 3-methylaminophenyl; acetamidophenyl such as 3-acetamidophenyl or pyridyl such as 3-pyridyl, 1-morpholinoethyl or benzyl.

More particular values for $R^4$ and $R^5$ together with the intervening nitrogen atom are pyrrolidino, dimethylamino, diethylamino, N-allyl-N-methylamino, N-isopropyl-N-methylamino, $\Delta^3$-pyrrolino or piperidino, especially pyrrolidino.

Preferred compounds of formula I and salts, especially pharmaceutically acceptable salts thereof by virtue of their potent analgesic activity are those wherein $R^1$ represents a halophenyl, dihalophenyl, nitrophenyl, cyanophenyl or trifluoromethylphenyl group especially a 3,4-dichlorophenyl or 4-trifluoromethylphenyl group. Other similarly preferred compounds of Formula I are those wherein X is a methylene group. Similarly preferred are compounds of formula I wherein $R^3$ represents isopropyl, isobutyl, sec.butyl, t-butyl, 1-($C_{1-4}$alkoxy)ethyl[for example 1-(t-butoxy)ethyl but especially 1-methoxyethyl], phenyl, hydroxyphenyl especially 4-hydroxyphenyl and 3-hydroxyphenyl, 1-methylthioethyl, 1-morpholinoethyl, dimethoxyphenyl, especially 3,4-dimethoxyphenyl, hydroxymethylphenyl especially 4-hydroxymethylphenyl, aminophenyl especially 3-aminophenyl, acetamidophenyl especially 3-acetamidophenyl or methylaminophenyl especially 3-methylaminophenyl. Similarly preferred compounds of formula I also include those wherein $R^4$ and $R^5$ together with the intervening nitrogen atom are a pyrrolidino, $\Delta^3$-pyrrolino or N-isopropyl-N-methylamino group. Especially preferred compounds of the present invention are those of the following Examples 19, 28, 54 and 94, these compounds being:-
(2S)-N-[2-(N-methyl-3,4-dichlorophenylacetamido)-2-phenylethyl]pyrrolidine,
(2S)-N-[2-(N-methyl-4-trifluoromethylphenylacetamido)-2-phenylethyl]pyrrolidine,
(2R,3R)-N-[2-(N-methyl-3,4-dichlorophenylacetamido)-3-methoxybutyl]pyrrolidine, and
(2S)-N-[2-(N-methyl-3,4-dichlorophenylacetamido)-2-phenylethyl] $\Delta^3$-pyrroline.
and the salts thereof, especially the pharmaceutically acceptable salts thereof.

Particular sub-groups of the compounds of the present invention of interest may be obtained by taking any one of the above-mentioned particular or preferred generic definitions for $R^1$, $R^2$, $R^3$, $R^4$, $R^5$ or X either singly or in combination with any other particular or preferred generic definition(s) for $R^1$, $R^2$, $R^3$, $R^4$, $R^5$ or X.

According to a further feature of the present invention there is provided a process for preparing the compounds of the present invention which comprises reacting a compound of formula IV (as set out hereinafter in which $R^1$ and X are as hereinbefore defined and Y represents an acid or an activated derivative thereof) with a compound of formula V (as set out hereinafter in which $R^2$, $R^3$, $R^4$ and $R^5$ are as hereinbefore defined) or a racemate thereof, optionally in protected form, and where necessary deprotecting the compound thus obtained to form a compound of formula I or racemate thereof and if desired reacting said compound or racemate with an acid to form a salt.

A compound of formula IV may for example be used in which Y represents an ester or an acid halide such as an acid chloride, acid anhydride or acyl imidazole.

Where the compound of formula IV used in an ester the reaction may advantageously be effected in the presence of an aprotic solvent or in the absence of a solvent. Such a reaction is preferably effected at a temperature of from ambient to the reflux temperature of the reaction mixture, but where no solvent is employed the reaction is preferably effected at a temperature no greater than 150 °C.

Where the compound of formula IV used is an acid chloride, an acid anhydride or an acyl imidazole the reaction is advantageously effected in the presence of an aprotic solvent, preferably at a temperature of from 0-60 °C.

The compound of formula V may for example be prepared by reaction of a compound of formula VI (wherein P represents a hydrogen atom or an amine protecting group and $R^3$, $R^4$ and $R^5$ are as hereinbefore defined) to convert said compound into a compound of formula V.

Such conversion may be effected in a number of different ways. Thus for example a compound of formula V in which $R^2$ is hydrogen may be prepared by reducing a compound of formula VI and where P in the compound of formula VI represents an amine protecting group, if necessary,subjecting the reduced compound obtained to deprotection. Alternatively where a compound of formula VI is used in which P represents an amine protecting group, the compound of formula VI may be subjected to deprotection prior to reduction.

Where it is desired to prepare a compound of formula V in which $R^2$ represents an alkyl group, the said compound may for example be prepared by reduction before or after alkylation or where alkylation is effected by reductive alkanoylation simultaneously with reduction. Alternatively where a compound of formula VI is used in which P represents an appropriate amine protecting group said compound may be reacted to convert the protecting group into the desired alkyl group.

Thus where it is desired to prepare a compound formula V in which $R^2$ is methyl, a compound of formula V in which $R^2$ is hydrogen may be subjected to methylation whereby to obtain the desired compound. Such a compound of formula V in which $R^2$ is methyl may also be prepared by reacting a compound of formula VI in which P represents an appropriate amine protecting group whereby to convert said amine protecting group into a methyl group. Such a reaction may for example be effected using a compound of formula VI in which P represents a benzyloxycarbonyl group, the reaction being effected by the use of a complex aluminium hydride reducing agent such as lithium aluminium hydride.

Where it is desired to prepare a compound of formula V in which $R^2$ is ethyl, n-propyl or isopropyl, a compound of formula V in which $R^2$ is hydrogen may be reacted with acetic or propionic acid or a reactive derivative thereof or with acetone, reduction being effected whereby to obtain the desired compound of formula V in which $R^2$ is ethyl, n-propyl or isopropyl.

The compound of formula VI may for example be prepared by reaction of a compound of formula VII (as set out hereinafter in which P, Y and $R^3$ are as hereinbefore defined) with a compound of formula VIII (as set out hereinafter in which $R^4$ and $R^5$ are as hereinbefore defined).

A compound of formula V may also be prepared by reduction of a compound of formula IX in which $R^2$, $R^3$, $R^4$ and $R^5$ are as hereinbefore defined preferably using a complex aluminium hydride reducing agent such as lithium aluminium hydride. The compound of formula IX is conveniently prepared by deprotecting a compound of formula X (in which $P^1$ represents an amine protecting group and $R^2$, $R^3$, $R^4$ and $R^5$ are as hereinbefore defined), by methods known per se. The compound of formula X may be prepared by reacting a compound of formula XI (in which $R^1$, $\overline{R^2}$, $\overline{R^3}$ and Y are as hereinbefore defined) with a compound of formula VIII. The compound of formula XI may be prepared by the amine protection of a compound of formula XII.

According to a further feature of the present invention there is provided a process for preparing a compound of the present invention in which $R^2$ is hydrogen, which process comprises selectively reducing a compound of formula III (as set out hereinafter wherein $R^1$, $R^3$, $R^4$, $R^5$ and X are as hereinbefore defined) or a racemate thereof optionally in protected form, and where necessary deprotecting the compound thus obtained to form a compound of formula I or racemate thereof; and if desired reacting said compound of formula I or racemate thereof with an acid to form a salt thereof.

The reduction may for example be effected by the use of a boron hydride reducing agent such as diborane. The reduction is conveniently effected in the presence of a solvent such as tetrahydrofuran.

The compound of formula III as hereinbefore defined may for example be prepared by reacting a compound of formula IV (as hereinbefore defined) with a compound of formula VI (as set out hereinafter in which P is hydrogen and $R^3$, $R^4$ and $R^5$ are as hereinbefore defined).

The compound of formula VI may for example be prepared by reacting a compound of formula VII with a compound of formula VIII and where P in the compound of formula VII represents an amine protecting group, deprotecting the compound of formula VI obtained to yield a compound of formula VI in which P is hydrogen.

According to a further feature of the present invention there is provided a process for the preparation of a compound of the present invention in which $R^3$ represents a moiety containing a free hydroxy group or a free amino group, which process comprises the hydrolysis of a corresponding compound in which $R^3$ represents a moiety containing an acyloxy or acylamino group whereby to obtain said compound of the present invention in which $R^3$ represents a moiety containing a free hydroxy group or a free amino group.

The starting material employed may for example correspond to a compound of the present invention except that $R^3$ represents a moiety containing the group $R^1$-X-CO-O or $R^1$-X-CO-NH-. The $R^1$-X-CO-O- or $R^1$-X-CO-NH-group may for example be introduced when reacting a compound of formula IV with a compound of formula V in which $R^3$ represents a moiety containing a free hydroxy or a free amino group, the reaction giving rise to diacylation.

If desired one compound of the present invention may be converted into another compound of the present invention by methods known per se.

Thus according to a further feature of the present invention there is provided a process for the preparation of a compound of the present invention (in which $R^3$ represent a moiety which contains a $C_{1-6}$ alkanoyloxy group) which process comprises the $C_{1-6}$ alkanoylation of a corresponding compound of the present invention in which $R^3$ represents a moiety which contains a hydroxy group.

The alkanoylation is conveniently effected by methods known per se such as the use of an an activated alkanoyl derivative such as an alkanoyl halide or ester, for example an acid chloride, acid anhydride or acyl imidazole, the alkanoylation being advantageously effected in a similar manner to that described above for the reaction of a compound of formula IV with a compound of formula V.

According to a further feature of the present invention there is provided a process for the preparation of compounds of the present invention in which X, $R^1$ and/or $R^3$ contains a $-SO_2-$ moiety, which process comprises oxidising a corresponding compound of the present invention in which X, $R^1$ and/or $R^3$ contains an -S- or -SO- moiety.

According to a further feature of the present invention there is provided a process for the preparation of compounds of the present invention in which X, $R^1$ and/or $R^3$ contains an -SO- moiety, which process comprises oxidising a corresponding compound of the present invention in which X, $R^1$ and/or $R^3$ contains an -S- moiety.

The oxidation of -S- or -SO- to $-SO_2$ and the oxidation of -S- to -SO- is conveniently effected by methods known per se for example by the use of hydrogen peroxide advantageously in the presence of acetic acid or a haloacetic acid for example trifluoroacetic acid, or a peroxy acid, for example 2-chloroperbenzoic acid, advantageously in the presence of an appropriate chlorinated solvent, for example dichloromethane.

According to a further feature of the present invention there is provided a process for the preparation of compounds of the present invention in which $R^1$ and/or $R^3$ contains a $C_{1-6}$ alkoxy, $C_{3-6}$ alkenyloxy or $C_{3-6}$ alkynyloxy group which process comprises alkenylating or alkynylating a corresponding compound of the present invention in which $R^1$ and/or $R^3$ contains a hydroxy group. The alkylation, alkenylation or alkynylation is conveniently effected by methods known per se for example by reaction with a haloalkane, haloalkene or haloalkyne.

According to a further feature of the present invention there is provided a process for preparing compounds of the present invention in which $R^1$ and/or $R^3$ represents a moiety containing an acylamino group which process comprises acylating a corresponding compound of the present invention in which $R^1$ and/or $R^3$ represent a moiety containing an amine group. The acylation is conveniently effected by methods known per se such as by the use of an activated acyl derivative such as an acyl halide or ester for example an acyl chloride, anhydride or imidazole, the acylation advantageously being effected in a similar manner to that described above for the reaction of a compound of formula IV with a compound of formula V.

It will be appreciated that, if desired, one $R^3$ group may be converted into another $R^3$ group at any stage in the preparation of the compounds of the present invention. Thus for example where aspartic acid is used as the starting material, the group corresponding to $R^3$ may, if desired, be reduced at any stage during the preparation of the compounds of the invention to yield the corresponding homoserine derivative. Similarly where a nitrophenylglycine is used as starting material the nitrophenyl moiety may if desired be reduced to the corresponding aminophenyl moiety at any stage during the preparation of the compounds of the invention.

The compounds used as starting materials in the above-mentioned processes may contain radicals which are not compatible with at least certain of the processes described as will readily be appreciated by those skilled in the art and in such cases it is desirable to protect the starting material(s) by the use of an appropriate protecting group prior to the relevant reaction. After completion of the reaction the protecting group may if desired be removed or if desired may be left intact for removal after formation of the

compound of formula I or racemate in protected form.

The relevant starting material may be in racemic form or in the desired optically active form. Where a racemate of the compound of formula I is obtained, the desired optically active enantiomer may be obtained by resolution according to conventional techniques. Where an optically active enantiomer of the compounds of formula I is obtained a racemate may if desired be obtained by racemisation according to conventional techniques.

Salts of the compounds of formula I or racemates thereof may if desired be prepared by reaction of the compound of formula I or racemate thereof with an appropriate acid, preferably an acid which affords a pharmaceutically acceptable anion for example a mineral acid such as hydrochloric acid or an organic acid such as fumaric or maleic acid.

Salts of the compound of formula I or racemate thereof may be converted to compounds of formula I or racemate thereof per se by conventional techniques, for example by ion exchange.

The compounds of the present invention possess analgesic activity which has been demonstrated in the writhing test which was conducted as follows:-

Groups of ten mice are given a sub-cutaneous (s.c.) injection of the test compound (0.2 ml/20 g bodyweight), thirty minutes prior to an intra-peritoneal (i.p.) injection of 0.4% acetic acid solution (0.4 ml mouse). The mice are then placed in individual observation boxes, and observed for the presence of an abdominal writhing response, the number of writhes being counted over a fifteen minute period, starting two minutes after the injection of acetic acid.

Four doses are needed for the calculation of the $ED_{50}$ dose (dose which inhibits the writhing response by 50%), unless the response curve is very steep, then three are acceptable. If the upper confidence limit is greater than $ED_{50}$ x 2, then the test is repeated. Compounds producing less then 50% inhibitiion of the writhing response at 10 mg/kg are considered inactive.

Compounds with an $ED_{50}$ <3 mg/kg are generally tested in the presence of naloxone. Naloxone is administered (3 mg/kg s.c., 0.2 ml/20 g body weight) simultaneously with the test compound i.e. 30 minutes prior to acetic acid. For a compound to be considered active in the writhing test the $ED_{50}$ should be <3 mg/kg s.c. and antagonism by naloxone (3 mg/kg should be >50%.

Compounds of the present invention have been found to possess diuretic and antiinflammatory activity.

The potency of a specific compound of the present invention depends upon its precise chemical structure, but generally speaking the compounds of the invention exhibit a potency in the range 0.001 to 10 mg/kg in the writhing test.

According to a further feature of the present invention therefore we provide pharmaceutical compositions comprising as active ingredient at least one compound of formula I or racemate thereof or a pharmaceutically acceptable salt of said compound or racemate in association with a pharmaceutically acceptable carrier or diluent.

The pharmaceutical compositions of the invention may be in a form suitable for oral, parenteral, topical or rectal administration. Thus, for example, they may be in orally-adminstrable unit dosage form, for example tablets or capsules, which may optionally be adapted for sustained release, or in injectable form, for example a sterile injectable solution or suspension, or in the form of a suppository for rectal administration or in a topically administrable form for antiiflamatory indications, for example an ointment or a nasal spray. The said pharmaceutical compositions may be produced by conventional methods using conventional diluents or carriers.

The compositions of the present invention may have veterinary as well as human applications, but where one of the compounds of the invention is used clinically in humans it is recommended to employ doses from 10 ug to 300 mg, for example 0.1 to 50 mg, once to three times per day, such a dosage range encompassing all routes of administration. No overt toxicity was noted at physiologically active doses.

According to a further feature of the present invention we provide a method of alleviating pain in warm-blooded animals which comprises administering to such animals an effective amount of a compound of formula I or a racemate or pharmaceutically acceptable salt of said compound or racemate.

The invention is illustrated by the following Examples in which the temperatures are expressed in degrees Celsius:-

**Example 1**

(2S)-N-[2-(N-Methyl-3,4-dichlorophenylacetamido)propyl]-pyrrolidine hydrochloride.

A solution of 3,4-dichlorophenylacetyl chloride (1.73 g) in dry dichloromethane (15 ml) was added to a solution of (2S)-N-(2-methylaminopropyl)pyrrolidine (1.0 g) in dry dichloromethane (10 ml). The solution was

kept at ambient temperature for 4 hours and then evaporated under reduced pressure to give a pale yellow oil. Trituration with ether gave 2.219 g of white solid, m.p. 167-169° which was filtered off and recrystallised from ethyl acetate (6.5 ml)/methanol (1.4 ml). This gave 1.463 g of a first crop of the desired compound in the form of the hydrated hydrochloride, m.p. 173-175°, and 0.407 g of a second crop, m.p. 171-173°, on further standing.

(2S)-N-(2-methylaminopropyl)pyrrolidine used as starting material was obtained as follows:-

(a) N-Benzyloxycarbonyl-(S)-alanine pyrrolidine

1-hydroxybenzotriazole (33.22 g) was added to a solution of N-benzyloxycarbonyl-(S)-alanine (50.0 g) in dry dichloromethane (400 ml) stirred under argon in an ice-bath. N,N′-Dicyclohexyl-carbodiimide (50.89 g) in dry dichloromethane (150 ml) was added to the mixture and the whole stirred for 1 hour. Pyrrolidine (17.46 g) was then added, the ice-bath removed and the mixture stirred at room temperature for 18 hours.

The mixture was filtered to remove dicyclohexylurea and the resulting solid washed with a little dichloromethane. A slower separation of a white solid occurred in the filtrate and 32.3g of this material, m.p. 127-130°, was collected. The remaining solution was evaporated under reduced pressure and the residue taken up in ethyl acetate (1400 ml). This was washed successively with saturated sodium bicarbonate solution (2 x 300 ml), water (300 ml) and brine (300 ml) and then dried over magnesium sulphate. Evaporation of the resulting solution under reduced pressure gave a white solid which was recrystallised from ethyl acetate (150 ml) to give 18.69 g of material, m.p. 126-128°. This was combined with the solid previously isolated and the whole recrystallised once more from ethyl acetate (250 ml) to give, in two crops, 41.97 g of N-benzyloxycarbonyl-(S)-alanine pyrrolidide, m.p. 128-131°.

(b) (2S)-N-(2-Methylaminopropyl)pyrrolidine

A solution of N-benzyloxycarbonyl-(S)-alanine pyrrolidide (20.70 g) in freshly distilled tetrahydrofuran (400 ml) was added dropwise over a period of 30 minutes to a stirred, ice-cooled suspension of lithium aluminium hydride (5.013 g) in freshly distilled tetrahydrofuran (100 ml) under argon. After the addition was complete, the ice-bath was removed and the mixture stirred at room temperature.

Saturated sodium carbonate solution was added dropwise to the mixture until effervescence ceased. The mixture was filtered through Celite and the filter cake washed thoroughly with ether. Evaporation of the filtrate gave an oil which was dissolved in ether (300 ml) and extracted with 2N hydrochloric acid (3 x 150 ml). The combined aqueous extracts were washed with ether (2 x 200 ml), then basified to >pH 11 with solid sodium hydroxide. The basified solution was extracted with ether (3 x 150 ml) and combined extracts dried over potassium carbonate.

Evaporation of the solvent under reduced pressure gave 5.40 g of (2S)-N-(2-methylaminopropyl)-pyrrolidine as a clear oil which could be used without further purification.

3,4-Dichlorophenylacetyl chloride used as starting material was obtained as follows:-

3,4-Dichlorophenylacetyl chloride

3,4-Dichlorophenylacetic acid (76.8 g) was dissolved in dry dichloromethane (300 ml) and oxalyl chloride (52 g, 36 ml) was added to the solution obtained in one portion. The reaction mixture was protected from atmospheric moisture with a calcium chloride guard tube and stirred mechanically whilst dimethylformamide (3 drops) was added. The solution was stirred at ambient temperature for 18 hours and then evaporated under reduced pressure to give a yellow oil. Distillation *in vacuo* afforded 80.0 g of 3,4-dichlorophenylacetyl chloride, b.pt 97-100°/0.6 mm, as a viscous pale yellow oil.

**Examples 2-53**

The following compounds of general formula I (wherein $R^2$ is methyl, X is $-CH_2-$, $-NR^4R^5$ is pyrrolidinyl and $R^1$ and $R^3$ are as defined below) were prepared in a similar manner to that described in Example 1. Thus, except as described hereinafter in relation to Example 51, 59 to 62 and 66 for the preparation of the compound of formula V and in relation to Examples 55, 57 and 58 for the preparation of the compounds of formula VI, compounds of formula VII (wherein Y is COOH, P represents a benzyloxycarbonyl group and $R^3$ is as defined below) are reacted with pyrrolidine to yield a compound of formula VI (wherein $-NR^4R^5$ represents a pyrrolidinyl group and $R^3$ is as defined below). The compound of formula VI obtained is reduced with lithium aluminium hydride to yield a compound of formula V (in which $R^2$ is methyl, $-NR^4R^5$ represents a pyrrolidinyl group and $R^3$ is as defined below). The indicated compound of formula I in each of Examples 2-69 was obtained by reaction of the compound of formula V with a compound of the formula $R^1$-X-COCl (in which X represents $-CH_2-$ and $R^1$ is as defined below).

| Compound No. | $R^1$ | $R^3$ | SALT | M.PT °C |
|---|---|---|---|---|
| 2 | 4-(trifluoromethyl)phenyl | methyl | HCl | 175-178 |
| 3 | 3,4-dichlorophenyl | benzyl | HCl. 0.5 $H_2O$ | 182-184 |
| 4 | 3,4-dichlorophenyl | isopropyl | HCl. $H_2O$ | 168-70 |
| 5 | 4-methoxyphenyl | isopropyl | HCl. 0.5 $H_2O$ | 221-224 |
| 6 | 3,4-dichlorophenyl | 2-(methylthio)ethyl | HCl | 174-177 |
| 7 | 4-methoxyphenyl | 2-(methylthio)ethyl | HCl | 155-158 |
| 8 | 3,4-dichlorophenyl | t-butoxymethyl | HCl | 160-162 |
| 9 | 3,4-dichlorophenyl | isobutyl | HCl | 174-176 |
| 10 | 4-methoxyphenyl | isobutyl | HCl | 179-181 |
| 11 | 4-(trifluoromethyl)phenyl | isobutyl | HCl | 189-190 |
| 12 | 4-bromophenyl | isobutyl | HCl | 207-209 |
| 13 | 3,4-dichlorophenyl | sec-butyl | HCl | 190-192 |
| 14 | 4-methoxyphenyl | sec-butyl | HCl | 183-185 |
| 15 | 4-(trifluoromethyl)phenyl | sec-butyl | HCl | 205-207 |
| 16 | 4-bromophenyl | sec-butyl | HCl | 202-205 |
| 17 | 4-(trifluoromethyl)phenyl | isopropyl | HCl. 0.5 $H_2O$ | 159-161 |

| Compound No. | $R^1$ | $R^3$ | Salt | M.PT °C |
|---|---|---|---|---|
| 18 | 3,4-dichlorophenyl | 1(R)-(t-butoxy)ethyl | HCl | 207-209 |
| 19 | 3,4-dichlorophenyl | phenyl | HCl | 233-235 |
| 20 | 3,4-dichlorophenyl | 4-hydroxyphenyl | HCl | 229-230 |
| 21 | 4-methylphenyl | isopropyl | HCl | 189-191 |
| 22 | 3,4-dichlorophenyl | 4-(t-butoxy)benzyl | HCl. $H_2O$ | 183-184 |
| 23 | phenyl | isopropyl | HCl | 162-164 |
| 24 | 3,4-dichlorophenyl | cyclohexylmethyl | HCl | 243-245 |
| 25 | 4-nitrophenyl | isopropyl | HCl. 0.5 $H_2O$ | 177-180 |
| 26 | 3,4-dichlorophenyl | 3-hydroxyphenyl | HCl | 228-230 |
| 27 | 4-nitrophenyl | 4-hydroxyphenyl | HCl | 232-234 |
| 28 | 4-(trifluoromethyl)phenyl | phenyl | HCl | 209-211 |
| 29 | 4-aminophenyl | methyl | HCl. 0.5 $H_2O$ | 250-251 |
| 30 | 4-(trifluoromethyl)phenyl | 3-nitrophenyl | HCl | 252-254 |
| 31 | 3,4-dichlorophenyl | 4-methylthiophenyl | HCl | 193-195 then 223-225 |
| 32 | 3,4-dichlorophenyl | t-butyl | HCl | 215-127(D) |

| Compound No. | $R^1$ | $R^3$ | Salt | M.PT °C |
|---|---|---|---|---|
| 33 | 4-bromophenyl | isopropyl | HCl | 203-206 |
| 34 | 2-chlorophenyl | isopropyl | HCl | 130-132 |
| 35 | 3-chlorophenyl | isopropyl | HCl.0.25 $H_2O$ | 150-153 |
| 36 | 4-chlorophenyl | isopropyl | HCl | 207-209 |
| 37 | 3,4-dichlorophenyl | n-propyl | HCl | 194-195 |
| 38 | 3,4-dichlorophenyl | ethyl | HCl | 218-219 |
| 39 | 3,4-difluorophenyl | isopropyl | maleate | 196-197 |
| 40 | 3-fluorophenyl | isopropyl | HCl | 165-166 |
| 41 | 4-fluorophenyl | isopropyl | HCl. 0.25 $H_2O$ | 175-178 |
| 42 | 4-cyanophenyl | isopropyl | maleate.0.5 $H_2O$ | 185-187 |
| 43 | 2-naphthyl | isopropyl | maleate | 174-175 |
| *44 | 3-quinolinyl | isopropyl | fumarate | 203-204 |
| 45 | 3-benzothienyl | isopropyl | HCl.0.5 $H_2O$ | 173-175 |
| **46 | 3-benzofuranyl | isopropyl | fumarate | 203-204 |
| 47 | 6-benzoxazolyl | isopropyl | maleate | 174-175 |
| 48 | 2-(1,3-dioxoisoindolinyl) | isopropyl | free base | 78-84 |
| 49 | 2-chloro-3-pyridyl | isopropyl | HCl | 157-159 |

*3-Quinolinyl acetic acid used as starting material in this example was obtained as in Acta.Chem.Scand. Ser.B. 1968, 22, page 2422-.

**3-Benzofuranyl acetic acid used as starting material in this example was obtained as in Chem.Pharm.Bull. 1982, 30, page 552-.

| Compound No. | $R^1$ | $R^3$ | Salt | M.PT °C |
|---|---|---|---|---|
| 50 | 2-chloro-5-pyridyl | isopropyl | HCl | 174-177 |
| 51 | 3,4-dichlorophenyl | 3-pyridyl | dioxalate | 86-87 |
| 52 | 3,4-dichlorophenyl | 3-methoxyphenyl | HCl | 221-222 |
| 53 | 3,4-dichlorophenyl | 4-methoxyphenyl | HCl | 222-223 |
| 54 | 3,4-dichlorophenyl | 1(R)-methoxyethyl | HCl. $0.5H_2O$ | 194-197 |
| 55 | 3,4-dichlorophenyl | 1(S)-methoxyethyl | HCl | 185-187 |
| 56 | 3,4-dichlorophenyl | methylthiomethyl | HCl. $0.5H_2O$ | 165-166 |
| 57 | 3,4-dichlorophenyl | 1(R)-methylthioethyl | HCl | 171-173 |
| 58 | 3,4-dichlorophenyl | 1(R)-morpholinoethyl | HCl | 228-230 |
| 59 | 4-nitrophenyl | 3,4-dimethoxyphenyl | HCl | 244-245 |
| 60 | 3,4-difluorophenyl | 3,4-dimethoxyphenyl | HCl, EtOH | 193-194 |
| 61 | 3,4-dichlorophenyl | 3,4-dimethoxyphenyl | HCl | 186-187 |
| 62 | 3,4-dichlorophenyl | 4-hydroxymethylphenyl | HCl | 215-218 |
| 63 | 3-benzisoxazolyl | isopropyl | maleate | 150-152 |
| 64 | 2-benzoxazolyl | isopropyl | maleate | 183-184 |
| 65 | 2-benzimidazolyl | isopropyl | maleate | 169-171 |
| 66 | 2-(5-fluoro)benzimidazolyl | isopropyl | fumarate $1.5H_2O$ | 168-169 |
| 67 | 3-(5-fluoro)benzisoxazolyl | isopropyl | maleate | 145-146 |
| 68 | 3-(6-fluoro)benzisoxazolyl | isopropyl | maleate | 135-136 |
| 69 | 3-(6-fluoro)benzisoxazolyl | 3,4-dimethoxyphenyl | maleate | 152-153 |

The compounds of Examples 6, 20, 26, 27, 30, 31, 32, 51 to 53, 60, 61, 62 and 69 were all obtained in racemic form. The compounds of Examples 8, 18 and 54 to 58 inclusive were obtained in R-stereoisomeric form. The compounds of the remaining Examples were obtained in the S-stereoisomeric form. (D) following a melting point means that the compound decomposed. The compound of formula V used as starting materials in the preparation of the compound of Example 51 and 59 to 62 inclusive and 66 were prepared

as detailed below, as were compounds of formula VI used as starting materials in the preparation of the compounds of Examples 55, 57 and 58. 2-(3-methoxyphenyl)glycine used as starting material in Example 52 was obtained as in Synthesis (1979), page 26. 2-Chloro-3-pyridyl acetic acid used as starting material in Example 49 was obtained in Journal of the American Chemical Society 1959, 81, pages 740-743. 2-Chloro-5-pyridyl acetic acid used as starting material in Example 50 was obtained as in Acta.Pharm.Suec. 1972, 9, pages 411-418.

(R,S)-N-[2-Methylamino-2-(3-pyridyl)ethyl]-pyrrolidine used as starting material in Example 51 was obtained as follows:-

(a) (R,S)-2-Methylamino-2-(3-pyridyl)acetic acid

3-Pyridine carboxaldehyde (8.96 g) and methylamine (10.0 ml of a 33% solution in ethanol) were dissolved in methanol (62.5 ml) at 0-5° and the solution treated dropwise over 20 minutes with trimethylsilylcyanide (9.9 g). The mixture was heated at 45° for 2 hours, cooled, evaporated to dryness, and the residue dissolved in concentrated hydrochloric acid (500 ml) and heated under reflux for 6 hours. The solution was evaporated to dryness, the residue taken up in water (200 ml) and the solution passed dropwise through an ion-exchange column (Dowex 50 W-X8, $H^+$ form). The column was washed with water until the washings were of neutral pH, then the product was eluted with 1N aqueous ammonia solution. The product-containing eluate was evaporated to dryness, the residue slurried with methanol, the precipitated solid filtered off, washed with methanol and dried to give (R,S)-2-methylamino-2-(3-pyridyl)acetic acid (5.4 g) mp 283-285°.

(b) (R,S)-N-[2-Methylamino-2-(3-pyridyl)acetyl]pyrrolidine

(R,S)-2-Methylamino-2-(3-pyridyl)acetic acid (2.5 g) was dissolved in a mixture of 1N sodium hydroxide solution (15 ml) and 2-methylpropan-2-ol (30 ml) at 50° and the solution treated dropwise over 10 minutes with di-tert-butyl dicarbonate (3.65g). The mixture was heated at 50° for 3 hours, evaporated to dryness, the residue dissolved in water (30ml) and washed with petrol ether (bp 60-80°) (3 x 25 ml). The aqueous layer was acidified with 1N citric acid solution (20ml) and extracted with ethyl acetate (10 x 25ml). The ethyl acetate extracts were combined, dried over sodium sulphate and evaporated.

The residue was dissolved in dry dichloromethane (75 ml) at 0-5° and 1-hydroxybenzotriazole (1.68 g) was added, followed by pyrrolidine (0.78 g), and the mixture stirred at 0-5° for 10 minutes. The mixture was treated with a solution of dicyclohexyl carboniimide (2.27 g) in dry dichloromethane (25 ml) at 0-5° over 5 minutes. The mixture was stirred at 5° for 16 hours, filtered and the filtrates evaporated to dryness. The residue was taken up in ethyl acetate (25 ml), washed with saturated sodium bicarbonate solution (2 x 15 ml), water (1 x 15 ml), the organic layer dried over sodium sulphate and evaporated.

The residue was dissolved in dry ethyl acetate (10 ml) and treated with 2N hydrogen chloride in dry ethyl acetate (7.5 ml) and the solution stirred at room temperature for 1 hour. The solution was evaporated to dryness, the residue dissolved in 5N sodium hydroxide solution (3 ml) and extracted with ethyl acetate (3 x 10 ml). The combined organic extracts were dried over sodium sulphate and evaporated. The residue was chromatographed on silica (Art 9385 230-400 mesh; 0.040-0.063 mm) and the product eluted with 10% methanol in chloroform. The product containing fractions were combined, evaporated to dryness and the residue crystallised from ethyl acetate/cyclohexane to give (R,S)-N-[2-methylamino-2-(3-pyridyl)acetyl]pyrrolidine (1.52 g) mp 105-106°.

(c) (R,S)-N-[2-Methylamino-2-(3-pyridyl)ethyl)]pyrrolidine

(R,S)-N-[2-Methylamino-2-(3-pyridyl)acetyl]pyrrolidine (1.0 g) was dissolved in dry tetrahydrofuran (50 ml) and added, over a period of 20 minutes to a suspension of lithium aluminium hydride (0.135 g) in dry tetrahydrofuran (50 ml) at 0-5° under argon. The mixture was stirred at 5°C for 3 hours, treated with water (0.135 ml), 15% aqueous sodium hydroxide solution (0.405 ml), water (0.135 ml), filtered and the filtrates evaporated to dryness to give 0.7g (RS)-N-[2-methylamino-2-(3-pyridyl)ethyl]pyrrolidine which was used without further purification.

(2S,3R)-N-(2-Benzyloxycarbonylamino-3-methoxybutyryl)pyrrolidine used as starting material in Example 55 was obtained as follows:-

a) Allothreonine O-methyl ether (0.88g) was dissolved in a solution of sodium hydroxide (0.29g) in water (10ml) and stirred at 0° whilst benzylchloroformate (1.03ml) was added over a 30 minute period. During this time the pH of the reaction mixture was maintained between 9.0 and 9.5 by the addition of 1N sodium hydroxide solution. After 2 1/2 hours at 0° an oily precipitate formed which was redissolved by the addition of dimethoxyethane (10ml) and stirring at 0° was continued for a further 5 hours. The reaction mixture was then washed with ether (2 x 50ml) and the aqueous phase acidified to pH2 by the addition of concentrated hydrochloric acid, then extracted with ethyl acetate (3 x 20ml). After backwashing with water and then brine (50ml each), the combined organic phase was dried over magnesium sulphate and evaporated under reduced pressure to give an oil (1.0g). This was dissolved in dry

dichloromethane (10ml) and cooled to 5°. N-hydroxybenzotriazole (0.66g) was added with stirring followed by dicyclohexylcarbodiimide. (0.885g) over 5 minutes and the reaction mixture stirred at 0-5° for 1 hour. Pyrrolidine (0.355ml) was added and stirring was continued for a further 24 hours at room temperature. Glacial acetic acid (0.5ml) was added and the resulting mixture allowed to stand for 3 days then filtered and the filtrate was diluted with dichloromethane (30ml). The solution so obtained was washed with saturated aqueous sodium bicarbonate solution (2 x 20ml), aqueous 2N hydrochloric acid (2 x 20ml), water and then brine (20ml each) and dried over magnesium sulphate, filtered and evaporated. The oil so obtained was subjected to dry flash chromatography on a bed of silica (ART7736 - 4cm deep, 7cm diameter) eluting with a gradient of 65% ethyl acetate in 60-80 petrol to 100% ethylacetate in 5% steps, 50ml elutions at each concentration to give (2S,3R)-N-(2-benzyloxycarbonylamino-3-methoxybutyryl)pyrrolidine (0.95g) as an oil.

b) Allothreonine O-methyl ether

Sodium hydride (8.3g, 55% dispersion in mineral oil) was suspended in dry tetrahydrofuran (70ml) and stirred under an argon atmosphere whilst a suspension of N-tritylallothreonine sodium salt (6.61g) in dry tetrahydrofuran (40ml) was added at -15° over 15 minutes. After stirring for a further 45 minutes at -15°, methyl iodide (2.4ml) was added and stirring was continued for 2 hours at -5°. An additional suspension of sodium hydride (8.3g, 55% dispersion in mineral oil) in dry tetrahydrofuran (100ml) was added, followed by methyl iodide (3.1ml) and the reaction mixture stirred for 24 hours at room temperature. Water (300ml) was added cautiously with cooling and the resulting solution extracted with ether (2 x 100ml). The aqueous phase was adjusted to pH6 with glacial acetic acid at less than 10°, then extracted with ether (6 x 50ml). The combined organic extract was backwashed with water (20ml), dried over sodium sulphate, filtered and evaporated to give a white foam (2.62g). This was treated with a 10% solution of glacial acetic acid in ethanol (20ml) and allowed to stand at room temperature for 2 days. The solid which precipitated was filtered off and the filtrate evaporated and partitioned between ether (50ml) and water (100ml). Evaporation of the aqueous phase gave a further solid, shown to be identical to the first by thin layer chromatography. The two solids were combined to give crude allothreonine O-methyl ether (0.94g), used directly in step (a).

c) N-Tritylallothreonine sodium salt

L-allothreonine (21.25g) was suspended in dry dichloromethane (315ml) and trimethylsilyl chloride (79.3ml) was added and the stirred reaction mixture was heated to reflux for 20 minutes. After cooling to 20°, a solution of triethylamine (87.1ml) in dry dichloromethane (180ml) was added and the mixture heated to reflux for 45 minutes then cooled to 0°. Anhydrous methanol (10.8ml) in dry dichloromethane (45ml) was then added dropwise and the mixture allowed to obtain room temperature. Triethylamine (24.9ml) followed by trityl chloride (49.8g) was added over a total of 15 minutes and the reaction mixture stirred for 24 hours at room temperature. Methanol (36.2ml) followed by triethylamine (24.9ml) was added and the mixture stirred for a further hour, then heated to reflux for 30 minutes. Evaporation of the solvent under reduced pressure gave an oily residue which was partitioned between aqueous 5% citric acid (900ml) and ether (900ml)/ethylacetate (500ml). 1N aqueous sodium hydroxide solution (360ml) was added to the organic layer and the solid which precipitated was filtered off and dried in vacuo to give N-tritylallothreonine sodium salt (23.2g)

(2R,3R)-(2-Benzyloxycarbonylamino-3-methylthiobutyryl)pyrrolidine used as starting material in Example 57 was obtained as follows:-

a) (2S,3S)-1-Benzyloxycarbonylamino-3-methyl-2-aziridinecarboxylic acid pyrrolidide (500mg) was dissolved in a solution of methanethiol (1.9g) in dry dichloromethane (20ml) at 0° and boron trifluoride etherate (0.5ml) added. The reaction mixture was left at 20° in a stoppered vessel and excess methanethiol removed by bubbling argon gas through the solution, then evaporating under reduced pressure. The residue was redissolved in dichloromethane (30ml), washed consecutively with a saturated aqueous solution of sodium bicarbonate, water and brine (30ml each), dried over magnesium sulphate and evaporated to give an oil. This was subjected to dry flash chromatography on a bed of silica (ART7736 - 3cm deep, 5cm diameter) eluting with a gradient of 50% 60-80 petrol in ethyl acetate to 100% ethyl acetate in 10% steps, 2 x 20 ml elutions at each concentration giving 12 fractions. Fractions 4-7 were combined and evaporated under reduced pressure to give (2R,3R)-(2-benzyloxycarbonylamino-3-methylthiobutyryl)pyrrolidine (280g) as an oil which was used directly in the preparation of the compound of Example 57.

b) (2S,3S)-1-Benzyloxycarbonylamino-3-methyl-2-aziridinecarboxylic acid pyrrolidide

(2S,3S)-3-Methyl-2-aziridinecarboxylic acid pyrrolidide (8.65g) was dissolved in dry chloroform (160ml) and cooled to 5°. Triethylamide (11.3ml) was added followed by benzyloxycarbonyl chloride (11.8ml) at 0-5° over 10 minutes with stirring. After 3 hours at 20° the solution was washed with a 10%

aqueous solution of citric acid (2 x 100ml), water and then brine (100ml each) and dried over magnesium sulphate. Evaporation under reduced pressure gave an oil which afforded a white solid on trituration with 60-80 petrol. Recrystallisation from ethyl acetate gave (2S,3S)-1-benzyloxycarbonylamine-3-methyl-2-aziridinecarboxylic acid pyrrolidide (11.2g) m.p. 116-117°.

c) (2S,3S)-3-Methyl-2-aziridinecarboxylic acid pyrrolidide.

Methyl (2S,3S)-1-trityl-3-methyl-2-aziridinecarboylate (35.6g), prepared by the method of K. Okawa and K. Nakajuna, Biopolymers, 20, pages 1811-1821 (1981), was dissolved in a mixture of chloroform (40ml) and methanol (20ml) and cooled to -10°. Trifluoroacetic acid (60ml) was added dropwise with stirring and the reaction mixture allowed to attain room temperature (20°) and left for 3 hours. Evaporation of the solvent under reduced pressure gave a residue which was dissolved in water (100ml) and adjusted to pH8 with solid sodium bicarbonate. After extraction with dichloromethane (6 x 100ml), the combined organic phases were evaporated to low bulk (40ml) under reduced pressure at 20° and methanol (50ml) added. The solution was slowly distilled through a Vigreux column (250mm) until the head temperature rose above 64°. The methanolic solution remaining in the distillation pot was treated with pyrrolidine (30ml), flushed with argon and heated to reflux under an argon atmosphere for 18 hours. After removal of the solvent in vacuo the residual oil was dissolved in ether and a small amount of insoluble material filtered off. Evaporation of the filtrate gave an oil which crystallised on standing to yield (2S,3S)-3-methyl-2-aziridinecarboxylic acid pyrrolidide (8.74g) m.p. 49-53°.

(2S,3R)-(2-t-Butyloxycarbonylamino-3-morpholinobutyryl)pyrrolidine used as starting material in Example 58 was obtained as follows:-

a) (2S,3S)-1-t-Butyloxycarbonylamino-3-methyl-2-aziridinecarboxylic acid pyrrolidide (1g) was dissolved in dry chloroform (3ml) and morpholine (3ml) added. After standing at 20° for 24 hours the solvent was evaporated and the residue heated to 70° for 18 hours on an oil bath. Excess morpholine was removed by evacuating at 0.5 mm Hg pressure for 4 hours and the residue triturated with methanol (5ml). The solid so formed was filtered off and the filtrate evaporated and redissolved in hot ethyl acetate (10ml) then cooled and filtered. The filtrate gave (2S,3R)-(2-t-butyloxycarbonylamino-3-morpholinobutyryl)-pyrrolidine (1.2g) as an oily solid after evaporation under reduced pressure.

b) (2S,3S)-1-t-Butyloxycarbonylamino-3-methyl-2-aziridinecarboxylic acid pyrrolidide.

(2S,3S)-3-Methyl-2-aziridinecarboxylic acid pyrrolidide (8.0g) was dissolved in 1,2-dimethoxyethane (160ml) and cooled to 0-5° and di-t-butyl dicarbonate (16.7g) in solution in 1,2-dimethyoxyethane (70ml) was added over 10 minutes with stirring. The reaction was allowed to attain room temperature (20°) and stirred for a further 2 1/2 hours. The reaction mixture was evaporated under reduced pressure to give an oil which was subjected to dry flash chromatography on a bed of silica (ART T136 - 10cm deep, 15cm diameter), eluting with a gradient of 10% ethyl acetate in 60-80 petrol to 100% ethylacetate in 10% steps, 100ml elutions at each concentration, to give (2S,3S)-1-t-butyoxycarbonylamino-3-methyl-2-aziridine carboxylic acid pyrrolidide (10.0g) as an oil, used directly in Stage (a)

(R,S)-N-[2-(3,4-Dimethoxyphenyl)-2-methylaminoethyl]pyrrolidine used as starting material in Examples 59-61 was obtained as follows:-

a) A solution of (R,S)-N-methoxycarbonyl-2-(3,4-dimethoxyphenyl)glycine pyrrolidide (4.1g; 0.0127mol) in tetrahydrofuran (33ml) was added dropwise to a stirred suspension of lithium aluminium hydride (1.44g; 0.037mol) in tetrahydrofuran (33ml) while cooling in an ice-bath. When the addition was complete the cooling bath was removed and the reaction mixture allowed to warm to ambient temperature. It was then heated at 54°C for 3 hours using an oil-bath.

After cooling, saturated aqueous sodium hydrogen carbonate solution was added dropwise until a white filterable precipiate was obtained. The aluminium salts were filtered off and the filtrate evaporated to dryness. The residue was dissolved in ethyl acetate (100ml) and the resulting solution washed with 2M aqueous hydrochloric acid (2 x 100ml). The combined aqueous extracts were washed with ethylacetate and then basified to pH11 with (30%) aqueous sodium hydroxide solution. The basic aqueous phase was extracted with ethylacetate (2 x 100ml). The combined organic extracts were washed, successively, with water and saturated aqueous sodium chloride solution and finally dried over magnesium sulphate. Evaporation to dryness gave (R,S)-N-[2-(3,4-dimethoxyphenyl)-2-methylaminoethyl]pyrrolidine as a pale yellow oil (3.5g).

b) (R,S)-N-Methoxycarbonyl-2-(3,4-dimethoxyphenyl)glycine pyrrolidide

A solution of (R,S)-N-methoxycarbonyl-2-(3,4-dimethoxyphenyl glycine (4.3g; 0.0158mol) in ethyl acetate (50ml) was treated with N,N′-carbonyldiimidazole (3.4g; 0.02mol) and the mixture stirred for one hour at ambient temperature. Pyrrolidine (1.7ml; 0.02mol) was added and the mixture stirred at ambient temperature overnight.

The reaction mixture was diluted with water and extracted with ether (2 x 100ml). The combined ether extracts were washed successively with 2M aqueous hydrochloric acid, water, saturated aqueous sodium chloride hydrogen carbonate solution, water and saturated aqueous sodium solution. The ether solution was dried over magnesium sulphate and evaporated to dryness to give (R,S)-N-methoxycarbonyl-2-(3,4-dimethoxyphenyl)pyrrolidide.

c) (R,S)-N-Methoxycarbonyl-2-(3,4-dimethoxyphenyl)glycine.

N-methoxycarbonyl-2-hydroxyglycine (11.0g; 0.06mol) was added to a mixture of glacial acetic acid (52ml) and concentrated sulphuric acid (5.8ml) and the resulting solution was stirred at ambient temperature undr an argon atmosphere. 1,2-Dimethoxybenzene (9.4ml; 0.073mol) was added portionwise, keeping the temperature below 25 °C, and the mixture was then stirred at ambient temperature overnight.

The reaction mixture was poured into a mixture of ice and water ( 300ml) and then extracted with ethylacetate (2 x 200ml). The combined organic extracts were washed, successively, with water and saturated aqueous sodium chloride and then finally dried over magnesium sulphate and evaporated to dryness to give a brown oil which crystallised on standing. Recrystallisation from ethanol gave 2(R,S)-N-methoxycarbonyl-2-(3,4-dimethoxyphenyl)glycine as a white crystalline solid (4.6g), m.p. 134-136 °C.

(R,S)-N-[2-(4-Hydroxymethylphenyl)-2-methylaminoethyl]pyrrolidine used as starting material in Example 62 was obtained as follows:-

a) from (R,S)-N-ethoxycarbonyl-2-(4-hydroxymethylphenyl)glycine pyrrolidide in a similar manner to that described in Examples 59-61 which was prepared from 2-(R,S)-N-ethoxycarbonyl-2-(4-hydroxymethylphenyl)glycine also in a similar manner to that described in Examples 59-61.

b) (R,S)-N-Ethoxycarbonyl-2-(4-hydroxymethylphenyl)glycine.

A suspension of 2(R,S)-N-ethoxycarbonyl-2-(4-chloromethylphenylglycine (13.0g; 0.048mol) in water (250ml) was made strongly alkaline by the addition of aqueous sodium hydroxide solution (30%; 10ml) and the resulting solution allowed to stand at ambient temperature overnight. A fine white solid precipitated and this was removed by filtration. The filtrate was acidified to pH3 with concentrated aqueous hydrochloric acid and the oily product extracted into ethylacetate (2 x 250ml). The combined ethyl acetate extracts were washed successively with water and saturated aqueous sodium chloride and then dried over magnesium sulphate and evaporated to give (R,S)-N-ethoxycarbonyl-2-(4-hydroxymethylphenyl) glycine as a pale yellow oil (8.8g).

c) (R,S)-N-Ethoxycarbonyl-2-(4-chloromethylphenyl)glycine.

A mixture of N-ethoxycarbonyl-2-hydroxyglycine (16.3g; 0.10mol) and benzyl chloride (52.8g; 0.40mol) dissolved in anhydrous methanesulphonic acid (100ml) was stirred at ambient temperature for 48 hours.

The reaction mixture was poured onto ice and then extracted with ethyl acetate (2 x 250ml). The combined organic extracts were washed 3 times with water followed by saturated aqueous sodium chloride solution. The organic phase was dried over magnesium sulphate and the solvent removed in vacuo. Further rotary evaporation under high vacuum was required to remove excess benzyl chloride.

The crude product was chromatographed on silica gel (Merck Keiselgel 9385; 200g), eluting with dichloromethane followed by choroform. The eluant contained further excess benzylchloride. The column was then eluted with a mixture of chloroform and methanol in the proportion (49:1), gradually increasing the proportion of methanol to (9:1). The fractions containing the product were combined and evaporated to give (R,S)-N-ethoxycarbonyl-2-(4-chloromethylphenyl)glycine as a clear oil (13.0g).

3-Benzisoxazolylacetic acid used as starting material in Example 63 was obtained as in e.g. Journal of Heterocyclic Chemistry, 1969, 6, page 279-. 2-Benzoxazolyl acetic acid used as starting material in Example 64 was obtained as in eg. Justus Liebig's Ann.Chem, 1939, 537, page 53-. 2-Benzimidazolylacetic acid used as starting material in Example 65 was obtained as in eg. Journal of the American Chemical Society, 1943, 65, page 1072-.

2-(5-Fluoro)benzimidazolylacetic acid used as starting material for the compound of Example 66 was prepared as follows:-

a) A solution of 2-(5-fluoro-benzimidazolylmethyl cyanide (1.15g) in conc $H_2SO_4$ (5.2ml) and water (5.2ml) was heated under reflux in an inert atmosphere for 3 hours. The pH of the cooled solution was then adjusted to about pH5.5 where the title compound crystallised in essentially quantitative yield m.p. 98-99 °C, resolidifies, re-melts 175-176 °C.

b) 2-(5-fluoro)benzimidazolylmethyl cyanide. An intimate mixture of 4-fluoro-1,2-phenylenediamine (5g) and cyanacetamido (6.7g) was placed in an oil bath at 180 °C and over a 15 minute period the temperature was raised to 210 °C. The mixture was then cooled and dissolved in ethyl acetate. The ethyl acetate solution was filtered, washed with water, dried and evaporated to dryness to yield a solid which

was recrystallised from aqueous methanol using decolourising charcoal. This solid was the above cyanide, m.p. 162-163 °C. 2-(5-fluoro)benzisoxazolylacetic acid used as starting material in Example 67 was obtained from 2-benzisoxaxolylacetic acid according to the method of Collect. Czech.Chem.Commun. 1964, 29, page 1035-. The starting acetic acid derivative of Examples 68 and 69 was likewise obtained.

**Example 70**

(R,S)-N-[2-(3,4-Dichlorophenylacetamido)-2-(3,4-dihydroxyphenyl)ethyl]pyrrolidine hydrobromide.
The compound of Example 61 vis. (RS)-N-[2-(3,4-dichlorophenylacetamido)-2-(3,4-dimethoxyphenyl)ethyl]-pyrrolidine hydrobromide (2.3g) was dissolved in dichloromethane (300ml) and stirred in an ice bath under an argon atmosphere. Boron tribromide solution (1M solution in dichloromethane, 9.3ml; 0.00934mol) was added and the mixture stirred at 0 °C for 1 hour. It was allowed to warm to room temperature and stirred overnight. Methanol (50ml) was added and the mixture stirred for 1 hour, and then evaporated to dryness. The residue was again treated with methanol (50ml) and re-evaporated. This process was repeated 3 times and finally the residue was crystallised from methanol to give (R,S)-N-[2-(3,4-dichlorophenylacetamido)-2-(3,4-dihydroxyphenyl)ethyl]pyrrolidine hydrobromide as white crystals, (1.5g) m.p. 234-235 °C.

**Example 71**

(2S)-N-[2-(N-Methyl-3,4-dichlorophenylacetamido)-4-hydroxybutyl]pyrrolidine oxalate

(2S)-N-[2-(N-Methyl-3,4-dichlorophenyl-acetamido)-4-(3,4-dichlorophenylacetoxy)butyl]pyrrolidine oxalate (1.73 g) [prepared as described below] was dissolved in methanol (100 ml) and a standardised aqueous solution of 1M sodium hydroxide (20.2 ml) added. The reaction mixture was stirred for 18 hours at room temperature and evaporated to dryness. The residue was partitioned between saturated aqueous sodium carbonate solution (50 ml) and ethyl acetate (50 ml) and after separating, the aqueous layer was washed with ethyl acetate (2 x 50 ml). After drying over sodium sulphate the combined organic layers were evaporated to give a gum, which was treated with oxalic acid (0.71 g) in ethyl acetate (25 ml). The resulting solid was filtered and recrystallised from ethyl acetate (30 ml) to give a white solid 2.2 g m.p. 134-136 °.

(2S)-N-[2-(N-Methyl-3,4-dichlorophenylacetamido)-4-(3,4-dichlorophenylacetoxy)butyl]pyrrolidine oxalate used as starting material was obtained as follows:-

(a) (2S)-(2-Methylamino-4-hydroxybutyl)-pyrrolidine.

(2S)-N-(2-Benzyloxycarbonylamino-3-t-butyloxycarbonylpropionyl)-pyrrolidine (5.36 g) was dissolved in dry tetrahydrofuran (THF) (70 ml) and added dropwise to a stirred suspension of lithium aluminium hydride (2.37 g) in THF (30 ml) over 15 minutes. After stirring at room temperature for 18 hours a further portion of lithium aluminium hydride (0.8 g) was added and the reaction mixture stirred for a further 24 hours. An argon atmosphere was maintained throughout. The reaction mixture was cooled to 0-5 ° and stirred whilst a saturated solution of sodium carbonate in water was added dropwise to destroy excess reducing agent. After the addition of ether (50 ml) and stirring for a further 5 minutes the precipitate so formed was filtered off and the filtrate evaporated to dryness. The residue was partitioned between 2N aqueous hydrochloric acid (70 ml) and ether (100 ml) and the aqueous layer separated, backwashed with ether (50 ml) then basified to pH13 by the addition of 30% aqueous sodium hydroxide solution and extracted with ether (4 x 50 ml). The final ether extracts were combined, dried over anhydrous potassium carbonate, filtered and evaporated to give an oil 1.8 g which was used without purification in the next stage.

b) (2S)-N-[2-(N-Methyl-3,4-dichlorophenylacetamido)-4-(3,4-dichlorophenylacetoxy)butyl]pyrrolidine oxalate.

(2S)-(2-Methylamino-4-hydroxybutyl)-pyrrolidine (1.73 g) was dissolved in dry dichloromethane (25 ml) and triethylamine (1.54 ml) added. The reaction mixture was stirred and cooled to 0-5 ° in an ice/water bath whilst 3,4-dichlorophenylacetyl chloride (4.72 g) was added dropwise in dichloromethane (5 ml) over 2 minutes. After a further 10 minutes the reaction mixture was allowed to attain room temperature and left for 2 hours, stirring throughout. The solvent was evaporated *in vacuo* and the residue partitioned between water (20 ml) and ethyl acetate (50 ml). Aqueous sodium hydroxide solution was added until the pH of the aqueous layer reached pH11.5. The organic layer was separated, backwashed with saturated aqueous sodium carbonate (50 ml), dried over sodium sulphate, filtered and evaporated to give a gum. This was subjected to flash chromatography on silica (Art 9385 230-400 mesh; 0.040-0.063 mm) eluting with aqueous ammonia (specific gravity 0.91)/ methanol/dichloromethane

(1/9/290) to give a brown gum. Treatment of this with oxalic acid (0.86g) in ethylacetate (30 ml) gave a crystalline white solid on standing 4.75 g m.p. 108.5-109.5.

## Example 72

(R,S)-N-[2-(N-Methyl-3,4-dichlorophenylacetamido)-3-(4-hydroxyphenyl)propyl]pyrrolidine hydrochloride

The compound of Example 22 (1.4 g) was suspended in 95% trifluoroacetic acid (25 ml) and stirred at room temperature for 2 hours. The reaction mixture was evaporated to low bulk and poured into a saturated aqueous solution of sodium bicarbonate (30 ml) and extracted with ether (3 x 30 ml). The combined organic phases were backwashed with water (30 ml), dried over magnesium sulphate, filtered and the evaporated residue was dissolved in ether (30 ml) and treated with ethereal hydrogen chloride to give a white precipitate, isolated by filtration 0.55 g m.p. 238-240°.

## Example 73

(2R,3R)-N-[2-(N-Methyl-3,4-dichlorophenylacetamido)-3-hydroxybutyl]pyrrolidine.

The compound of Example 18 (0.45 g) was dissolved in 10% aqueous trifluoroacetic acid and stood at room temperature for 1 hour. The reaction mixture was evaporated to low bulk and basified by the addition of aqueous 2N sodium hydroxide solution (15 ml), then extracted with ether (3 x 20 ml). The combined organic extracts were dried over sodium sulphate, filtered and evaporated to give an oil which crystallised on standing. Trituration with n-pentane/ethylacetate (10 ml; 20/1) gave the title free base as a white crystalline solid 0.24 g m.p. 98-100°.

## Example 74

(2R,S)-N-[2-(N-Methyl-3,4-dichlorophenylacetamido)-4-methylsulphinylbutyl]pyrrolidine oxalate

A stirred solution of the compound of Example 6 (0.425 g) in dry dichloromethane (10 ml) was cooled to -30° and a solution of 85% m-chloroperbenzoic acid (0.218 g) in dry dichloromethane (2 ml) added dropwise. Stirring was maintained for a further 0.5 hr at -30° and the reaction mixture allowed to warm to ambient temperature. After washing with a saturated aqueous solution of sodium bicarbonate (4 x 10 ml) the aqueous extracts were backwashed with dichloromethane (3 x 10 ml) and the combined organic layers dried over sodium sulphate, filtered and evaporated to give a colourless viscous oil. This was treated with oxalic acid (0.102 g) in ethyl acetate (10 ml) and the oily deposit formed was triturated with ethyl acetate (10 ml) at -50°. The solid so obtained was filtered off (0.3 g) m.p. 118-121° (decomposition).

## Example 75

(R,S)-N-[2-(N-Methyl-3,4-dichlorophenylacetamido)-2-(3-aminophenyl)-ethyl]pyrrolidine hydrochloride

(R,S)-N-[2-(N-Methyl-3,4-dichlorophenylacetamido)-2-(3,4-dichlorophenylacetamido)phenyl-ethyl] pyrrolidine (prepared as described below) (34 g) was refluxed in 20:80 water: ethanol (by volume) (200 ml) containing sodium hydroxide pellets (20 g) overnight. The mixture was evaporated under reduced pressure to remove most of the ethanol and then extracted with ethyl acetate. The organic layer was washed with water and then brine and finally dried ($MgSO_4$) and evaporated to give an oil, yield : 21.0 g.

A sample with ethereal hydrogen chloride gave a white solid (dihydrochloride) m.p. 248-9°. (methanol/ethyl acetate).

(R,S)-N-[2-(N-Methyl-3,4-dichlorophenylacetamido)-2-(3,4-dichlorophenylacetamido)phenyl-ethyl]-pyrrolidine used as starting material was obtained as follows:-

a) (R,S)-N-(2-methoxycarbonylamino-2-(3-aminophenylacetyl)pyrrolidine.

N-Methoxycarbonyl-(R,S)-3-nitrophenylglycine pyrrolidide (15.0 g) [prepared by protecting 3-nitrophenyl-glycine in conventional manner and subsequent reaction of the protected compound with pyrrolidine in similar manner to that described in Example 1a] was dissolved in glacial acetic acid (200 ml). 3-nitrophenylglycine may be prepared by the method of Sriid and Kjaer, Acta. Chim. Scand. 1963, 17, page 2394.

The catalyst (10% Pd/C) (2.0 g) was added and the mixture was stirred at room temperature under a hydrogen atmosphere until uptake of the gas was complete. The hydrogen atmosphere was flushed away with argon and the mixture filtered through celite to give a clear solution. This was evaporated and then azeotroped several times with toluene to remove the last traces of acetic acid to give an oil which slowly crystallised (14.5 g) m.p. 147-9° (isopropanol).

b) (R,S)-N-[2-(N-methyl-3,4-dichlorophenylacetamido)2-(3,4-dichlorophenylacetamido)phenyl-ethyl]-pyrrolidine.

(R,S)-N-(2-methoxycarbonylamino-2-(3-amino-phenylacetyl)pyrrolidine (14.3 g) was dissolved in dichloromethane (1200 ml) and 3,4-dichlorophenylacetyl chloride (29.2 g) was added slowly with ice cooling to the stirred solution. After completion of the addition the mixture was stirred for 3 hours at room temperature. The solvent was evaporated and the residue was triturated with ether and filtered to give an off white solid 34.0 g m.p. 249-251° (methanol/ethyl acetate).

**Example 76**

(2S)-N-[2-(N-Methyl-3,4-dichlorophenylacetamido)-4-acetoxybutyl]pyrrolidine hydrochloride

A stirred solution of the free base of the compound of Example 67 (0.5 g) in dichloromethane (10 ml) was cooled to +5° and treated with acetyl chloride (111 µl). The solution was allowed to warm to room temperature and then stirred overnight. The solvent was evaporated in vacuo and the residue triturated with ether. The resulting solid was collected and washed with ether. The crude product was purified by recrystallisation from ethyl acetate containing a trace of methanol. Yield (0.42 g) mp 170-171°.

**Example 77**

(R,S)-N-[2-(N-Methyl-3,4-dichlorophenylacetamido)-2-(4-methylsulphonylphenyl)ethyl]pyrrolidine hydrochloride

A stirred solution of the compound of Example 31 (0.6 g) in anhydrous trifluoroacetic acid (2.5 ml) was cooled to +5° using an ice/water bath. This solution was treated with peroxytrifluoroacetic acid (0.67 ml) prepared in the manner described in J. Org. Chem. 47, 3774-7, (1982) using 30% hydrogen peroxide (8.6 ml) and trifluoroacetic acid (25 ml). The mixture was stirred at room temperature overnight and then evaporated to dryness. The crude residue was purified using column chromatography ("Merck Kieselgel 9385" eluting with 9:1 dichloromethane/methanol containing 1% aqueous ammonia solution (specific gravity 0.880). Evaporation of the combined fractions containing the pure product gave an oil which was crystallised as the hydrochloride salt in ether by addition of ethereal hydrogen chloride. (Yield 0.32 g) mp 216-218°.

**Example 78**

(R,S)-N-[2-(N-Methyl-3,4-dichlorophenylacetamido)-2-(4-methylsulphinylphenyl)ethyl]pyrrolidine hydrochloride

Using a procedure similar to that described in Example 77 , the compound of Example 31 (0.6 g) was treated with peroxytrifluoroacetic acid (prepared as in Example 60) (0.33 ml) at +5° and stirred for 2 hours. The crude product was purified as in Example 77 to give a white solid (0.35 g) mp 210-212°.

**Example 79**

(R,S)-N-[2-(N-Methyl-3,4-dichlorophenylacetamido)-2-(3-propoxyphenyl)ethyl]pyrrolidine hydrochloride

The free base of the compound described in Example 26 (0.488 g) in 2 ml toluene and 5 ml DMF was stirred for 18 hours at ambient temperature with 0.207 g anhydrous potassium carbonate and 255 mg 1-iodopropane, and then for 5 hours at 60°. the mixture was diluted with water and the product extracted with ethyl acetate. After washing, drying and evaporating 0.42 g of a pale yellow oil was obtained. This was dissolved in isopropanol and treated with ethereal HCl. Evaporation gave a solid which was recrystallised from ethyl acetate/ether mp 146° (yield 0.14 g).

## Example 80

(R,S)-N-[2-(N-Methyl-3,4-dichlorophenylacetamido)-2-(3-acetamidophenyl)ethyl]pyrrolidine hydrochloride.

A stirred solution of the free base of the compound of Example 75 (0.4 g) in dichloromethane (5 ml) was treated with a solution of acetyl chloride (0.1 ml) in dichloromethane (2 ml) with ice-cooling. The mixture was stirred for one hour at room temperature and evaporated to dryness. The solid residue was recrystallised from ethyl acetate containing methanol to give an amorphous solid, 0.25 g mp 231-2°.

## Examples 81-90

The following compounds of general formula I (wherein $R^1$ is 3,4-dichlorophenyl, $R^2$ is methyl, X is $-CH_2-$ and $-NR^4R^5$ and $R^3$ are as defined below) were prepared in a similar manner to that described in Example 1. Thus compounds of formula VII (wherein Y is COOH, P represents a benzyloxycarbonyl group and $R^3$ is as defined below) are reacted with the amine $R^4R^5NH$ (in which $-NR^4R^5$ is as defined below to yield a compound of formula VI (wherein $-NR^4R^5$ and $R^3$ are as defined below). The compound of formula VI obtained is reduced with lithium aluminium hydride to yield a compound of formula V (in which $R^2$ is methyl and $-NR^4R^5$ and $R^3$ are as defined below). The compound of formula V thus obtained is then reacted with a compound of formula $R^1$-X-COCl (in which X represents $-CH_2-$ and $R^1$ is 3,4-dichlorophenyl) to yield the indicated compound of formula I.

| Compound No. | $R^3$ | $NR^4R^5$ | Salt | M.PT °C |
|---|---|---|---|---|
| 81 | phenyl | diethylamino | HCl | 190-192 |
| 82 | phenyl | piperidino | HCl | 232-233 |
| 83 | isopropyl | piperidino | HCl | 173-175 |
| 84 | isopropyl | dimethylamino | HCl | 175-177 |
| 85 | isopropyl | diethylamino | free base | 85-87 |
| 86 | phenyl | dimethylamino | HCl | 231-232 |
| 87 | methyl | dimethylamino | HCl | 204-206 |
| 88 | methyl | N-allyl-N-methylamino | HCl | 148-151 |
| 89 | isopropyl | N-isopropyl-N-methylamino | Fumarate | 164-165 |
| 90 | phenyl | N-allyl-N-methylamino | HCl | 169-171 |

N-Isopropylmethylamine, used in the synthesis of Example 89, was prepared according to the method of N.A. Shaik, H.Oelschlaeger, and D. Rothley; Arch.Pharm. 314, 644-646, (1981).

The compounds of Example 81-90 were all obtained in the S-stereoisomeric form.

**Examples 91-94**

(wherein $R^2$ is methyl, X is $-CH_2-$, $-NR^4R^5$ is delta$^3$-pyrrolinyl and $R^1$ and $R^3$ are as defined below) were prepared in a similar manner to that described in Example 1. Thus compounds of formula VII (wherein Y is COOH, P represents a benzyloxycarbonyl group and $R^3$ is as defined below) are reacted with delta$^3$-pyrroline to yield a compound of formula VI (wherein $-NR^4R^5$ represents a delta$^3$-pyrrolinyl group and $R^3$ is as defined below). The compound of formula VI obtained is reduced with lithium aluminium hydride to yield a compound of formula (in which $R^2$ is methyl, $-NR^4R^5$ represents a delta$^3$-pyrrolinyl group and $R^3$ is as defined below). The compound of formula V thus obtained in then reacted with a compound of formula $R^1$-XCOCl (in which X represents $-CH_2-$ and $R^1$ is as defined below) to yield the indicated compound of formula I.

| Compound No. | $R^1$ | $R^3$ | Salt | M.PT °C |
|---|---|---|---|---|
| 91 | 3,4-dichlorophenyl | methyl | HCl | 208-210 |
| 92 | 4(trifluoromethyl)phenyl | methyl | HCl | 221-223 |
| 93 | 4-bromophenyl | methyl | HCl | 199-202 |
| 94 | 3,4-dichlorophenyl | phenyl | HCl | 214-216 |

All the compounds of Examples 91-94 were obtained in the S-stereoisomeric form.

**Example 95**

(2S)-N-[2-(3,4-Dichlorophenylacetamido)-3-methylbutyl]-pyrrolidine hydrochloride (2S)-N-[2-(3,4-dichlorophenylacetamido)-3-methylbutyryl]pyrrolidine (residue from (b) below) was taken up in dry tetrahydrofuran (36 ml) and treated with a 1M solution of borane in tetrahydrofuran (24.0 ml) under argon at room temperature over 20 minutes. The mixture was heated under reflux for 3 hours, cooled to room temperature, treated with methanol (25 ml) over 5 minutes, and heated under reflux for 4 hours. The solution was evaporated to dryness, the residue chromatographed on alumina (Woelm N-32-63) and eluted with 50% ethyl acetate in petrol ether (b.p. 60-80°). The product containing fractions were combined, evaporated to dryness, the residue treated with ethereal HCl, evaporated to dryness and the residue crystallised from methanol/ethyl acetate to give (2S)-N-[2-(3,4-dichlorophenylacetamido)-3-methylbutyl]-pyrrolidine hydrochloride, 0.25 g, m.p. 189-190°.

(2S)-N-[2-(3,4-dichlorophenylacetamido)-3-methylbutyryl]pyrrolidine used as starting material was obtained as follows:-

(a) (2S)-N-(2-amino-3-methylbutyryl)pyrrolidine hydrochloride

N-t-butoxycarbonyl-S-valine (43.4 g ) was dissolved in dry dichloromethane (500 ml) at 0-5° and 1-hydroxybenztriazole (33.7 g) was added to the solution. Pyrrolidine (15.6 g) was added and the mixture stirred at 0-5° for 10 minutes, then treated with a solution of dicyclohexylcarbodiimide (45.3 g) in dry dichloromethane (250 ml) over 15 minutes. The mixture was stirred at 5° for 16 hours, filtered and the filtrates evaporated to dryness. The residue was taken up in ethyl acetate (250 ml), washed with water (1 x 150 ml), saturated sodium bicarbonate solution (2 x 150 ml), 1N citric acid solution (1 x 100 ml), water (1 x 200 ml), the organic layer dried over sodium sulphate and evaporated. The residue was treated with 2N HCl in ethyl acetate (300 ml) and stirred at room temperature for one hour. The solution was evaporated to dryness and the residue crystallied from methanol-ethyl acetate to give (2S)-N-(2-amino-3-methylbutyryl)pyrrolidine hydrochloride, 28.2 g, m.p. 177°-179°.

(b) (2S)-N-[2-(3,4-dichlorophenylacetamido)-3-methylbutyryl]pyrrolidine

(2S)-N-(2-amino-3-methylbutyryl)pyrrolidine hydrochloride (1.61 g) and triethylamine (1.74 g) were dissolved in dry dichloromethane (20 ml) at 0-5°. The mixture was treated with a solution of 3,4-dichlorophenylacetyl chloride (1.92 g) in dry dichloromethane (20 ml) at 0-5° over 10 minutes. The mixture was stirred at room temperature for 1 hour, evaporated to dryness, the residue taken up in water (20 ml) and extracted with ethyl acetate (3 x 20 ml). The combined organic extracts were washed with 1N aqueous hydrochloric acid (1 x 20 ml), water (1 x 20 ml), saturated sodium bicarbonate solution (2 x 20 ml), water (2 x 20 ml), brine (1 x 20 ml), the organic layer dried over magnesium sulphate and evaporated to give 2.6 g (2S)-N-[2-(3,4-dichlorophenylacetamido)-3-methylbutyryl]pyrrolidine.

**Example 96**

(2S)-N-[2-(N-Ethyl-3,4-dichlorophenylacetamido)-3-methylbutyl]pyrrolidine oxalate.

(2S)-N-(2-Ethylamino-3-methylbutyl)pyrrolidine (residue from (a) below) was dissolved in dry dichloromethane (10 ml) at 0-5° and treated with a solution of 3,4-dichloro phenylacetyl chloride (0.89 g) in dry dichloromethane (10 ml) at 0-5° over 5 minutes. The mixture was stirred at room temperature for 1 hour, evaporated to dryness, the residue taken up in water (10 ml) and extracted with ethyl acetate (3 x 10 ml). The combined organic extracts were washed with saturated sodium bicarbonate solution (2 x 10 ml), water (2 x 10 ml) brine (1 x 10 ml), the organic layer dried over sodium sulphate and evaporated. The residue was chromatographed on alumina (Woelm N-32-63) and eluted with 10% ethyl acetate in petrol ether (b.p. 60°-80°). The product containing fractions were combined, evaporated to dryness, the residue treated with ethereal oxalic acid, evaporated to dryness and the residue crystallised from methanol/ethyl acetate to give 0.56 g (2S)-N-[2-(N-Ethyl-3,4-dichlorophenylacetamido)-3-methylbutyl]pyrrolidine oxalate. m.p. 166-167°.

(a) (2S)-N-(2-Ethylamino-3-methylbutyl)pyrrolidine used as starting material was obtained as follows:-
(2S)-N-(2-amino-3-methylbutyryl)pyrrolidine hydrochloride (2.07 g) and triethylamine (2.24 g) were dissolved in dry dichloromethane (25 ml) at 0-5°. The mixture was treated with a solution of acetyl chloride (0.86 g) in dry dichloromethane (10 ml) at 0-5° over 10 minutes. The mixture was stirred at room temperature for 1 hour, evaporated to dryness, the residue taken up in water (25 ml) and extracted with ethyl acetate (3 x 25 ml). The combined organic extracts were washed with 1N aqueous hydrochloric acid (1 x 25 ml), water (1 x 25 ml), saturated sodium bicarbonate solution (2 x 25 ml), water (2 x 25 ml), brine (1 x 25 ml), the organic layer dried over magnesium sulphate and evaporated. The residue was taken up in dry tetrahydrofuran (20 ml) and added over 10 minutes to a suspension of lithium aluminium hydride (0.3 g) in dry tetrahydrofuran (20 ml) at 0-5° under argon. The mixture was stirred at room temperature under argon for 16 hours, treated with water (0.3 ml), 15% aqueous sodium hydroxide solution (0.9 ml), water (0.3 ml), filtered, and the filtrates evaporated to dryness to give 0.67 g (2S)-N-(2-Ethylamino-3-methylbutyl)pyrrolidine.

**Example 97**

(2S)-N-[2-(N-Isopropyl-3,4-dichlorophenylacetamido)-3-methylbutyl]pyrrolidine.

(2S)-N-(2-Isopropylamino-3-methylbutyl)pyrrolidine (residue from (b) below) was taken up in dry dichloromethane (20 ml) at 0-5° and treated with a solution of 3,4-dichlorophenyl acetyl chloride (1.75 g) in dry dichloromethane (20 ml) at 0-5° over 10 minutes. The mixture was stirred at room temperature for 1 hour, evaporated to dryness, the residue taken up in water (20 ml) and extracted with ethyl acetate (3 x 20 ml). The combined organic extracts were washed with saturated sodium bicarbonate solution (2 x 20 ml), water (2 x 20 ml), brine (1 x 20 ml), the organic layer dried over sodium sulphate and evaporated. The residue was chromatographed on alumina (Woelm N32-63) and eluted with 5% ethyl acetate in petrol ether (b.p. 60°-80°). The product containing fractions were combined and evaporated to give 0.45 g (2S)-N-[2-(N-Isopropyl-3,4-dichlorophenylacetamido)-3-methylbutyl]pyrrolidine $(M + H)^+ = 385$.

(2S)-N-(2-Isopropylamino-3-methylbutyl)-pyrrolidine used as starting material was obtained as follows:-

(a) (2S)-N-(2-Amino-3-methylbutyl)pyrrolidine

(2S)-N-(2-Amino-3-methylbutyryl)pyrrolidine hydrochloride (41.3 g) was dissolved in water (200 ml). The solution was treated with 5N sodium hydroxide solution (44 ml) and extracted with ethyl acetate (3 x 250 ml). The combined organic extracts were dried over sodium sulphate and evaporated. The residue was taken up in dry tetrahydrofuran (200 ml) and added over 1.5 hours to a suspension of lithium aluminium hydride (7.5 g) in dry tetrahydrofuran (200 ml) at 0-5° under argon. The mixture was stirred at room temperature for 16 hours, treated with water (7.5 ml), 15% aqueous sodium hydroxide solution

(22.5 ml), water (7.5 ml), filtered, and the filtrates evaporated to dryness. The residue was distilled to give 10.0 g (2S)-N-(2-amino-3-methylbutyl)pyrrolidine b.p. = 83-85° at 7.5 mm.

(b) (2S)-N-(2-Isopropylamino-3-methylbutyl)pyrrolidine

(2S)-N-(2-Amino-3-methylbutyl)pyrrolidine (1.0 g) and dry acetone (0.72 g) were dissolved in dry tetrahydrofuran (10 ml) at 0-5°. The solution was treated with a 1M solution of borane in tetrahydrofuran (7.1 ml) under argon at 0-5° over 10 minutes. The mixture was stirred at room temperature for 16 hours, treated with methanol (10 ml) over 5 minutes and the mixture heated under reflux for 16 hours. The solution was evaporated to dryness to give 1.17 g (2S)-N-(2-isopropylamino-3-methylbutyl)pyrrolidine as a crude oil.

## Examples 98-103

The following compounds of general formula I (wherein $R^2$ is methyl, $R^3$ is isopropyl, $-NR^4R^5$ is pyrrolidinyl and $R^1$ and X are as defined below) were prepared in a similar manner to that described in Example 1. Thus compounds of formula VII (wherein Y is COOH, P represents a benzyloxycarbonyl group and $R^3$ is isopropyl) are reacted with pyrrolidine to yield a compound of formula VI (wherein $-NR^4R^5$ represents a pyrrolidinyl group and $R^3$ is isopropyl). The compound of formula VI obtained is reduced with lithium aluminium hydride to yield a compound of the formula V (in which $R^2$ is methyl, $-NR^4R^5$ represents a pyrrolidinyl group and $R^3$ is isopropyl). The compound of formula V thus obtained is then reacted with a compound of formula $R^1$-X-COCl (in wich X and $R^1$ are as defined below) to yield the indicated compound of formula I.

| Compound No. | $R^1$ | X | Sa | M.PT °C |
|---|---|---|---|---|
| 98 | 3,4-dichlorophenyl | $OCH_2$ | HCl.$_2$O | 103-104 |
| 99 | 4-bromophenyl | $OCH_2$ | HCl | 172-174 |
| 100 | 3-(trifluoromethyl)phenyl | $OCH_2$ | HCl | 157-160 |
| 101 | 4-nitrophenyl | $OCH_2$ | HCl. | $(M+H)^+$ = 350 |
| 102 | 4-fluorophenyl | $OCH_2$ | HCl.$H_2O$ | 128-130 |
| 103 | 2-methoxyphenyl | $OCH_2$ | HCl. | 126-128 |
| 104 | 4-cyanophenyl | $OCH_2$ | HCl. | (softens 75-80) 182-185 |
| 105 | 3,4-dichlorophenyl | $SCH_2$ | HCl | 174-176 |
| 106 | 3,4-dichlorophenyl | $CH_2CH_2$ | HCl | 181-182 |

All the compounds of Examples 98 to 106 were obtained ie S-stereoisomeric form.

compounds of the formula $R^1$-X-$CO_2$H used as starting materials in Examples 99-101 and 104-106 were obtained according to the method of the following references:-

(99) - Coll.Czech.Chem.Comm., 1967, 32, page 1197.
(100) - Journal of the American Chemical Society, 1947, 69, page 718.
(101) - Journal of the Chemical Society, 1922, 121, page 1591.
(104) - Pharmazie, 1976,31(8), pages 528-532
(105) - Journal of Medicinal Chemistry, 1972, 15(9), page 940.
(106) - Canadian Journal of Chemistry, 1960, 38, page 2042.

## Example 107

(R,S)-N-[2-(N-Methyl-3,4-dichlorophenylacetamido)-2-(3-methylaminophenyl)ethyl]pyrrolidine hydrochloride

2(R,S)-N-[2-Methylamino-2-(3-methylaminophenyl)ethyl]pyrrolidine (1.0g), (0.0043m) was dissolved in methylene chloride (50ml) and 3,4-dichlorophenylacetyl chloride (2.3g, 2.2 equivalents) in methylene chloride (50ml) was added dropwise while cooling in an ice bath. The mixture was then stirred at 5°C for a

further 15 minutes and then for one hour at room temperature. The mixture was evaporated, triturated with ether and filtered to give the crude bis adduct (2.8g).

This material was treated with 10% potassium hydroxide and extracted with ethyl acetate. The organic layer was separated dried with magnesium sulphate and evaporated to give the free base. The was columned on Merck 7734 silica using 5% methanol/methylene chloride to give the pure product 1.6g (58%).

The above material was dissolved in water (10ml)/ethanol (40ml) containing potassium hydroxide pellets (5.0g). The mixture was then refluxed overnight, cooled and evaporated. It was then extracted with ethyl acetate, dried with magnesium sulphate and evaporated to give a gum. This was dissolved in ether and etheral hydrogen chloride was added to give a precipitate. This was filtered off but rapidly changed to a gum (hygroscopic). The liquor and gum were recombined and evaporated. The residue was dissolved in hot ethyl acetate/methanol and filtered. This solution was left to stand to give a deposit of pinkish crystals (310mg), yield 15% overall, as based on the starting pyrrolidine material, found to be pure by TLC, 20% methanol/$CH_2Cl_2$ + $NH_3$. After one further recrystallisation m.p. 242-4° dec. the following analysis was determined:-

| | C | H | N |
|---|---|---|---|
| Found | 53.2 | 6.1 | 8.3 |
| Theory for | 53.5 | 5.9 | 8.5 |
| $C_{22}H_{27}ON_3Cl_2$ 2HCl(493). | | | |

(R,S)-N-[2-Methylamino-2-(3-methylaminophenyl)ethyl]pyrrolidine used as starting material was obtained as follows:-

a) (R,S)-N-Methyoxycarbonyl-2-(3-methoxycarbonylaminophenyl)glycine]pyrrolidide

(R,S)-(3-aminophenyl)glycine pyrrolidide (2.9g, 0.0104m) was dissolved with stirring in methylene chloride (30ml) and triethylamine (1.53ml 0.011M) was added. The mixture was cooled in an ice bath and methyl chloroformate (0.85ml 0.011M) was added. Stirring was then continued at room temperature overnight. The solution was evaporated and partitioned between ethyl acetate and water. The organic layer was washed with water then dilute hydrochloric acid and finally water, dried with magnesium sulphate and evaporated to give 1.7g (49%) of a foam. This could be further purified by columning on Merck silica 7734 using 75% ethyl acetate/25% methylene chloride.

b) (R,S)-N-[2-Methylamino-2-(3-methylaminophenyl)ethyl pyrrolidine

The product from a) above (2.0g 0.006M) was stirred at reflux under argon with lithium aluminium hydride (1.5g 0.04m) in dry tetrahydrofuran (100ml) overnight. The cooled solution was then treated with saturated sodium carbonate solution to destroy the excess lithium aluminium hydride, filtered and the tetrahydrofuran evaporated to give the product oil (1.0g, 72% yield). Using TLC, 20% methanol/methylene chloride and one drop of ammonia the product was found to be essentially pure and was used without further purification in the next stage.

The Octanol-water distribution coefficient referred to hereinbefore may be determined as follows:-

The compound for test is dissolved (1 mg/50 ml) in buffer (0.01M Phosphate, pH 7.4) presaturated with the organic solvent. The u.v. spectrum is recorded to ensure that the u.v. absorbance is between 0.3 and 1.5 units for at least one band in the spectrum, preferably at $\lambda > 250$ nm. The solution is filtered to remove excess solid material and transferred (5 ml) by pipette into a stoppered test tube. The required volume of octanol, presaturated with water at the appropriate pH, is then transferred, again by pipette into the test tube. Ideally, the ratio of aqueous to organic should be such that an absorbance change of fifty per cent is achieved, although a minimum aqueous volume of 3 ml is required. The stoppered test tube is then vigorously shaken for several minutes and allowed to stand for fifteen minutes. The mixture is transferred by pipette into a centrifuge tube and placed in the centrifuge opposite a second tube containing an equal volume of water and spun at 3600 r.p.m. for ten minutes. The centrifuge tube is removed and the upper layer is carefully removed by pasteur pipette. A second centrifugation may be necessary after this process to reseparate the layers. Using a second, clean pasteur pipette placed to the bottom of the centrifuge tube with application of positive pressure, the aqueous layer (2-3 ml) is transferred to the cuvette taking care not to carry over droplets of the organic layer. The u.v.spectrum of this solution is taken overlaid onto the u.v. spectrum of the starting aqueous stock solution, both referenced against aqueous buffer. The spectrum of

the stock solution should be compared with the original spectrum to ensure no degradation of the compound has occurred during the experiment.
The distribution coefficient, D, is calculated using the following equation -

$$D = \frac{A_{B,w} - A_{A,w}}{A_{A,w}} \times \frac{V_w}{V_o}$$

$A_{B,w}$ = Absorbance in aqueous phase before addition of organic layer
$A_{A,w}$ = Absorbance in aqueous phase after addition of organic layer
$V_w$ = Volume of the aqueous phase
$V_o$ = Volume of organic phase

The result must be the same whatever the wavelength chosen. In all cases the measurement is confirmed by repeating the experiment by partitioning from the organic solvent into water. The result of the reverse experiment can be calculated by the following expression -

$$D = \frac{A_{A,o}}{A_{B,o} - A_{A,o}} \times \frac{V_w}{V_o}$$

$A_{A,o}$ = Absorbance in organic phase before addition of buffer
$A_{B,o}$ = Absorbance in organic phase after addition of buffer.

## Pharmaceutical Composition Examples

### Example A

Tablets:

Each tablet contains:

| | |
|---|---|
| (2S)-N-[2-(N-methyl-3,4-dichlorophenylacetamido)-2-phenylethyl]$\Delta^3$pyrroline | 0.1mg |
| Dicalcium phosphate | 42mg |
| Methylcellulose, U.S.P (15 c.p.s) | 1.6mg |
| Lactose | 6mg |
| Calcium Stearate | 0.5mg |

The active ingredient and dicalcium phosphate are mixed well, granulated with a 7.5% aqueous solution of mthylcullulose, passeed through a No. 8 screen and dried. The granulate obtained is then passed through a No. 12 screen, mixed with the remaining ingredients (lactose and calcium stearate) and compressed to yield tablets each weighing approximately 50mg.

**Example B**

Capsules:

Each capsule contains:

| | |
|---|---|
| Compound of the present invention | 0.2mg |
| Lactose U.S.P. | 47mg |
| Starch U.S.P | 2.5mg |
| Calcium stearate | 0.35mg |

The above-mentioned ingredients are obtained in finely powdered form, mixed thoroughly and then filled into appropriately sized hard gelatin capsules.

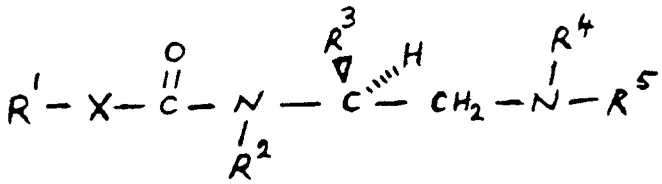

I

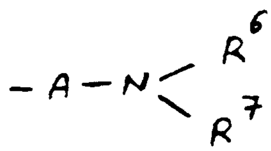

II

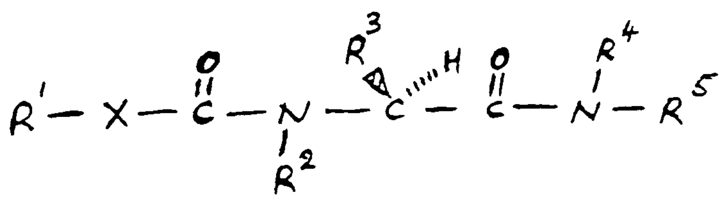

III

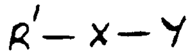

IV

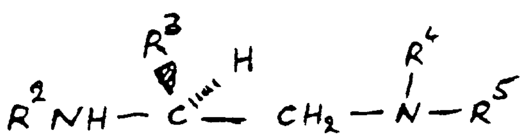

V

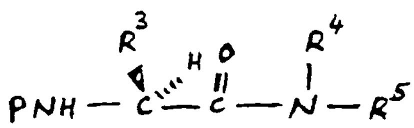

VI

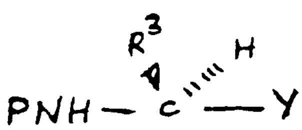

VII

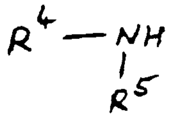

VIII

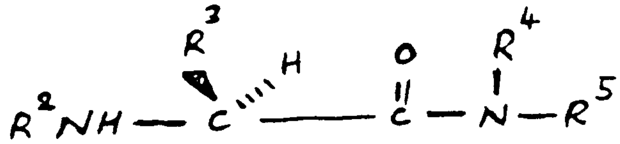

IX

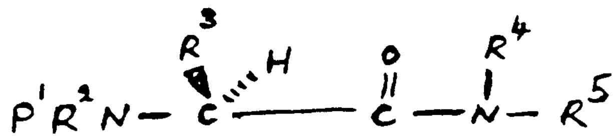

X

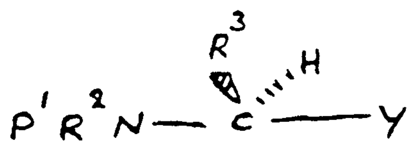

XI

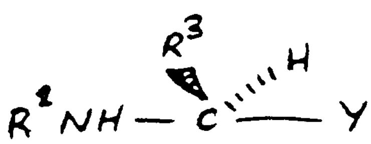

XII

## Claims

**Claims for the following Contracting States : BE, CH, DE, FR, GB, IT, LI, NL**

**1.** A compound of the formula I

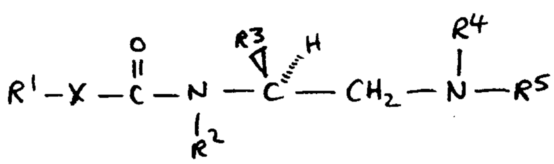

wherein $R^1$ represents a $C_{6-10}$ aryl group optionally substituted by one, two or three substituents independently selected from halogen, hydroxy, trifluoromethyl, cyano, nitro, amino, aminocarbonyl, carboxy, sulphonic acid, ($C_{1-6}$ alkoxy)carbonyl, $C_{1-6}$ alkyl sulphide, $C_{1-6}$ alkyl sulphoxide, $C_{1-6}$ alkyl sulphone, $C_{1-6}$ alkanoyl, $C_{1-6}$ alkoxy, $C_{3-6}$ alkenyloxy, $C_{3-6}$ alkynyloxy, $C_{1-6}$ acylamino, $C_{1-6}$ acylmethylamino, $C_{1-6}$ alkyl, $C_{1-6}$ monoalkylamino, ($C_{1-6}$ monoalkylamino)carbonyl, and a group of formula II

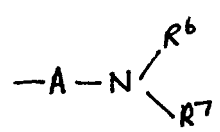

in which A is -CO- or a single bond and $R^6$ and $R^7$ which may be the same or different each represent a $C_{1-6}$ alkyl group or $R^6$ and $R^7$ together with the intervening nitrogen atom represents a cyclic amine with 4 to 7 ring atoms and where appropriate the oxides thereof), or $R^1$ represents a heterocyclic

moiety comprising a 5-or 6- membered heterocyclic ring containing one or two heteroatoms independently selected from oxygen, nitrogen and sulphur, the ring optionally being fused with a benzene ring and the heterocyclic and/or benzene ring being optionally substituted on carbon by one or more substituents selected from amino, halogen, hydroxy (and keto-tautomers thereof) $C_{1-6}$ alkyl, $C_{1-6}$ alkoxy, $C_{3-6}$ alkenyloxy and $C_{3-6}$ alkynyloxy any nitrogen heteroatom optionally carrying an oxygen atom or a hydroxy or $C_{1-3}$ alkyl group;

X represents a single bond, $-CH_2-$, $-OCH_2-$, $-SCH_2-$, $-SOCH_2-$, $-SO_2CH_2-$ or $-CH_2-CH_2-$;

$R^2$ represents hydrogen or $C_{1-3}$ alkyl;

$R^3$ represents an alkyl, cycloalkyl or cycloalkylmethyl group having up to 7 carbon atoms, the cycloalkyl moiety where present, having 3 to 6 carbon atoms, said group optionally being substituted by one or more substituents selected from hydroxy, amino, amidino, guanidino, aminocarbonyl, carboxy, $C_{1-6}$ alkoxy, ($C_{1-6}$ alkoxy)carbonyl, ($C_{3-6}$ alkenyloxy)carbonyl, ($C_{3-6}$ alkynyloxy)carbonyl, $C_{1-6}$ alkanoyloxy, $C_{1-6}$ alkylsulphide, $C_{1-6}$ alkylsulphoxide, $C_{1-6}$ alkylsulphone, $C_{1-6}$ -(monoalkylamino)carbonyl, $C_{1-6}$ acylamino, $C_{1-6}$ acylmethylamino, $C_{1-6}$ monoalkylamino, a group of formula II;

or $R^3$ represents the group $-B-R^1_a$ in which B represents $-CH_2-$, $-CH(CH_3)-$ or a single bond and $R^1_a$ represents an optionally substituted $C_{6-10}$ carbocyclic aryl group as defined for $R^1$;

or $R^3$ represents the group $-D-R_b$ in which D represents a single bond, $-CH_2-$, $-CH(CH_3)-$, $-CH_2-O-$, $-CH(CH_3)-O-$, $-CH_2-S-$, $-CH(CH_3)-S-$, $-CH_2-NH-$ or $-CH(CH_3)-NH-$ and $R_b$ represents a 4-to 6-membered heterocyclic ring containing up to 4 heteroatoms selected from oxygen, sulphur and nitrogen, the heterocyclic ring optionally being substituted on nitrogen or sulphur by oxygen or on nitrogen by hydroxy or $C_{1-3}$ alkyl and/or the ring optionally being substituted on carbon by one or more substituents selected from amino, hydroxy, thio (and their tautomers), cyano, halogen, $C_{1-3}$ alkoxy, $C_{1-3}$ monoalkylamino, $C_{1-3}$ acylamino, $C_{1-3}$ acylmethylamino, $C_{1-3}$ alkylthio and the group of formula II as herein defined;

and $R^4$ and $R^5$, which may be the same or different, each represents a $C_{3-5}$ alkenyl, $C_{3-5}$ alkynyl, $C_{1-6}$ alkyl, or $C_{4-7}$ cycloalkylalkyl group;

or $R^4$ and $R^5$ together with the intervening nitrogen atom represent a 4-7-membered heterocyclic ring which optionally contains a further heteroatom selected from oxygen and sulphur] or a racemate thereof, and the salts of said compound or racemate.

**2.** A compound as claimed in claim 1 wherein $R^1$ represents a phenyl or naphthyl group substituted by one or two substituents selected from halogen, trifluoromethyl, cyano, nitro, $C_{1-6}$alkyl or $C_{1-6}$alkoxy; or $R^1$ is a 5- or 6-membered heterocyclic ring containing one or two heteroatoms selected from oxygen, nitrogen or sulphur, the heterocyclic ring being fused with a benzene ring, and the heterocyclic ring and/or benzene ring being optionally substituted by halogen and/or hydroxy, linkage to the remainder of the compound of formula I being via the heterocyclic ring.

**3.** A compound as claimed in claim 1 wherein $R^1$ represents a halophenyl, dihalophenyl, trifluoromethylphenyl, methoxyphenyl, methylphenyl, nitrophenyl, cyanophenyl, naphthyl, benzothienyl, benzofuranyl, benzoxazolyl, benzisoxazolyl optionally substituted by fluorine, benzimidazolyl optionally substituted by fluorine or 2-(1,3-dioxoisoindolinyl) group.

**4.** A compound as claimed in any one of claims 1 to 3 wherein $R^1$ represents a halophenyl, dihalophenyl, nitrophenyl, cyanophenyl or trifluoromethylphenyl group.

**5.** A compound as claimed in any one of the previous claims wherein $R^1$ represents a 3,4-dichlorophenyl or 4-trifluoromethylphenyl group.

**6.** A compound as claimed in any one of claims 1 to 5 having a distribution coefficient between octanol and aqueous buffer of 1 or greater at pH 7.4.

**7.** A compound as claimed in any one of the preceding claims wherein X represents $-CH_2-$.

**8.** A compound as claimed in any one of the preceding claims wherein $R^2$ represents methyl.

**9.** A compound as claimed in any one of the preceding claims wherein $R^3$ represents an alkyl group having up to 7 carbon atoms optionally being substituted by hydroxy, $C_{1-6}$ alkylsulphide, $C_{1-6}$

alkylsulfinyl or $C_{1-6}$ alkoxy; or $R^3$ represents a phenyl group optionally substituted by one or two substituents selected from hydroxy, nitro, $C_{1-6}$ alkylsulphide, $C_{1-6}$ alkysulfinyl, $C_{1-6}$ alkylsulphonyl, $C_{1-6}$ alkoxy, amino,
$C_{1-6}$ alkylamino or $C_{1-6}$ acylamino or $R^3$ represents a group of the formula $-D-R_6$ in which D represents a single bond or a - $CH(CH_3)$-group and $R_6$ represents a 5- or 6-membered heterocyclic ring containing one or two heteroatoms selected from oxygen, nitrogen or sulphur, or $R^3$ represents a benzyl group.

**10.** A compound as claimed in any one of the preceding claims wherein $R^3$ represents an isopropyl, isobutyl,
sec-butyl, t-butyl, 1-($C_{1-4}$alkoxy)ethyl, phenyl, hydroxyphenyl, 1-methylthioethyl, 1-morpholinoethyl, dimethoxyphenyl, hydroxymethylphenyl, aminophenyl, acetamidophenyl or methylaminophenyl group.

**11.** A compound as claimed in any one of the preceding claims wherein $R^4$ and $R^5$ together with the intervening nitrogen atom represents a pyrrolidino, piperidino, $\Delta^3$-pyrrolino, dimethylamino, diethylamino, N-allyl-N-methylamino or N- isopropyl-N-methylamino group.

**12.** A compound as claimed in any one of the proceding claims wherein $R^4$ and $R^5$ together with the intervening nitrogen atom represent a pyrrolidino, $\Delta^3$-pyrrolino or N-isopropyl-N- methylamino group.

**13.** A compound of formula I selected from
(2S)-N-[2-(N-methyl-3,4-dichlorophenylacetamido)-2-phenylethyl]pyrrolidine,
(2S)-N-[2-(N-methyl-4-trifluoromethylphenylacetamido)-2-phenylethyl]pyrrolidine,
(2S,3R)-N-[2-(N-methyl-3,4-dichlorophenylacetamido)-3-methoxybutyl]pyrrolidine, and
(2S)-N-[2-(N-methyl-3,4-dichlorophenylacetamido)-2-phenylethyl]$\Delta^3$-pyrroline and the salts thereof.

**14.** A compound as claimed in any one of the preceding claims in the form of a pharmaceutically acceptable salt thereof.

**15.** A process for preparing a compound of the formula I as defined in claim 1 or a salt thereof, which comprises:-
a) reacting a compound of the formula IV

$R^1$-X-Y

(in which $R^1$ and X are as hereinbefore defined and Y represents an acid or an activated derivative thereof), with a compound of formula V

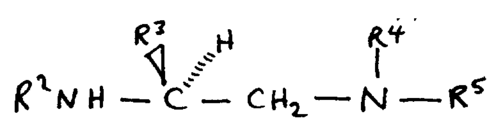

(in which $R^2$, $R^3$, $R^4$ and $R^5$ are as hereinbefore defined) or a racemate thereof optionally in protected form, and where necessary deprotecting the compound thus obtained to form a compound of formula I or racemate thereof
b) for the preparation of a compound of formula I in which $R^2$ is hydrogen, selectively reducing a compound of formula III,

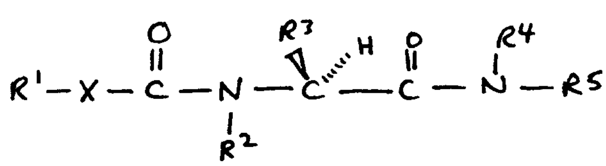

wherein $R^1$,$R^3$,$R^4$,$R^5$ and X are as hereinbefore defined, or a racemate thereof optionally in protected

form and where neccesary deprotecting the compound thus obtained to form a compound of formula I or racemate thereof

c) for the preparation of a compound of formula I in which $R^3$ represents a moiety containing one or more free hydroxy groups or a free amino group, hydrolysing a corresponding compound in which $R^3$ represents a moiety containing one or more acyloxy or alkoxy groups or an acylamino group.

d) for the preparation of a compound of formula I in which $R^3$ represents a moiety which contains the $C_{1-6}$ alkanoyloxy group, the selective $C_{1-6}$ alkanoylation of a corresponding compound of the present invention in which $R^3$ represents a moiety which contains a hydroxy group

e) for the preparation of a compound of formula I in which X, $R^1$ and/or $R^3$ contains a $-SO_2$-moiety, oxidising a corresponding compound of the present invention in which X, $R^1$ and/or $R^3$ contains an -S- or $-SO_2$-moiety

f) for the preparation of a compound of formula I in which X, $R^1$ and/or $R^3$ contains a -SO- moiety, oxidising a corresponding compound of the present invention in which X, $R^1$ and/or $R^3$ contains an -S- moiety

g) for the preparation of a compound of formula I in which $R^1$ and/or $R^3$ contains a $C_{1-6}$ alkoxy, $C_{3-6}$ alkenyloxy or $C_{3-6}$ alkynyloxy group, alkylating, alkenylating or alkynylating a corresponding compound of the present invention in which $R^1$ and/or $R^3$ contains a hydroxy group

h) for the preparation of a compound of formula I in which $R^1$ and/or $R^3$ represents a moiety containing an acylamino group, acylating a corresponding compound of the present invention in which $R^1$ and/or $R^3$ represent a moiety containing an amine group

and, whereafter, when the compound of formula I or a racemate thereof is obtained in the form of a base and a salt is required, reacting said compound of formula I obtained with an acid to form the salt.

**16.** A pharmaceutical composition comprising as active ingredient at least one compound of formula 1 as defined in claim 1 or racemate thereof or a pharmaceutically acceptable salt of said compound or racemate in association with a pharmaceutically acceptable carrier or diluent.

**Claims for the following Contracting State : ES**

**1.** A process for preparing a compound of the formula I

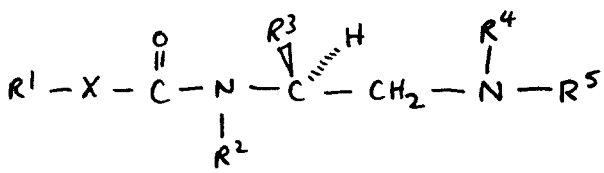

wherein $R^1$ represents a $C_{6-10}$ aryl group optionally substituted by one, two or three substituents independently selected from halogen, hydroxy, trifluoromethyl, cyano, nitro, amino, aminocarbonyl, carboxy, sulphonic acid, ($C_{1-6}$ alkoxy)carbonyl, $C_{1-6}$ alkyl sulphide, $C_{1-6}$ alkyl sulphoxide, $C_{1-6}$ alkyl sulphone, $C_{1-6}$ alkanoyl, $C_{1-6}$ alkoxy, $C_{3-6}$ alkenyloxy, $C_{3-6}$ alkynyloxy, $C_{1-6}$ acylamino, $C_{1-6}$ acylmethylamino, $C_{1-6}$ alkyl, $C_{1-6}$ monoalkylamino, ($C_{1-6}$ monoalkylamino)carbonyl, and a group of formula II

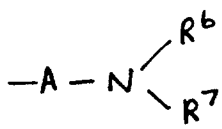

in which A is -CO- or a single bond and $R^6$ and $R^7$ which may be the same or different each represent a $C_{1-6}$ alkyl group or $R^6$ and $R^7$ together with the intervening nitrogen atom represents a cyclic amine with 4 to 7 ring atoms and where appropriate the oxides thereof), or $R^1$ represents a heterocyclic moiety comprising a 5-or 6- membered heterocyclic ring containing one or two heteroatoms independently selected from oxygen, nitrogen and sulphur, the ring optionally being fused with a benzene ring and the heterocyclic and/or benzene ring being optionally substituted on carbon by one or more substituents selected from amino, halogen, hydroxy (and keto-tautomers thereof) $C_{1-6}$ alkyl, $C_{1-6}$

alkoxy, $C_{3-6}$ alkenyloxy and $C_{3-6}$ alkynyloxy any nitrogen heteroatom optionally carrying an oxygen atom or a hydroxy or $C_{1-3}$ alkyl group;

X represents a single bond, $-CH_2-$, $-OCH_2-$, $-SCH_2-$, $-SOCH_2-$, $-SO_2CH_2-$ or $-CH_2-CH_2-$;

$R^2$ represents hydrogen or $C_{1-3}$ alkyl;

$R^3$ represents an alkyl, cycloalkyl or cycloalkylmethyl group having up to 7 carbon atoms, the cycloalkyl moiety where present, having 3 to 6 carbon atoms, said group optionally being substituted by one or more substituents selected from hydroxy, amino, amidino, guanidino, aminocarbonyl, carboxy, $C_{1-6}$ alkoxy,

($C_{1-6}$ alkoxy)carbonyl, ($C_{3-6}$ alkenyloxy)carbonyl, ($C_{3-6}$ alkynyloxy)carbonyl, $C_{1-6}$ alkanoyloxy, $C_{1-6}$ alkylsulphide, $C_{1-6}$ alkylsulphoxide, $C_{1-6}$ alkylsulphone, $C_{1-6}$ (monoalkylamino)carbonyl, $C_{1-6}$ acylamino, $C_{1-6}$ acylmethylamino, $C_{1-6}$ monoalkylamino, a group of formula II (as herein defined);

or $R^3$ represents the group $-B-R^1{}_a$ in which B represents $-CH_2-$, $-CH(CH_3)-$ or a single bond and $R^1{}_a$ represents an optionally substituted $C_{6-10}$ carbocyclic aryl group as defined for $R^1$;

or $R^3$ represents the group $-D-R_b$ in which D represents a single bond, $-CH_2-$, $-CH(CH_3)-$, $-CH_2-O-$, $-CH(CH_3)-O-$, $-CH_2-S-$, $-CH(CH_3)-S-$, $-CH_2-NH-$ or $-CH(CH_3)-NH-$ and $R_b$ represents a 4-to 6-membered heterocyclic ring containing up to 4 heteroatoms selected from oxygen, sulphur and nitrogen, the heterocyclic ring optionally being substituted on nitrogen or sulphur by oxygen or on nitrogen by hydroxy or $C_{1-3}$ alkyl and/or the ring optionally being substituted on carbon by one or more substituents selected from amino, hydroxy, thio (and their tautomers), cyano, halogen, $C_{1-3}$ alkoxy, $C_{1-3}$ monoalkylamino, $C_{1-3}$ acylamino, $C_{1-3}$ acylmethylamino, $C_{1-3}$ alkylthio and the group of formula II as herein defined;

and $R^4$ and $R^5$, which may be the same or different, each represents a $C_{3-5}$ alkenyl, $C_{3-5}$ alkynyl, $C_{1-6}$ alkyl, or $C_{4-7}$ cycloalkylalkyl group;

or $R^4$ and $R^5$ together with the intervening nitrogen atom represent a 4-7-membered heterocyclic ring which optionally contains a further heteroatom selected from oxygen and sulphur] or a racemate thereof, and the salts of said compound or racemate, which comprises:-

a) reacting a compound of the formula IV

$R^1-X-Y$

(in which $R^1$ and X are as hereinbefore defined and Y represents an acid or an activated derivative thereof), with a compound of formula V (in which $R^2$, $R^3$, $R^4$ and $R^5$ are as hereinbefore defined) or a racemate thereof, optionally in protected form, and where necessary deprotecting the compound thus obtained to form a compound of formula I or racemate thereof

b) for the preparation of a compound of formula I in which $R^2$ is hydrogen, selectively reducing a compound of formula III,

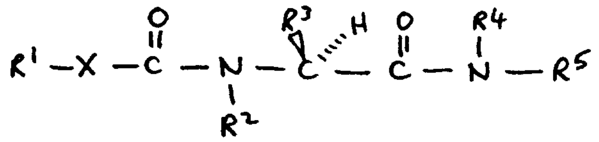

wherein $R^1$,$R^3$,$R^4$,$R^5$ and X are as hereinbefore defined, or a racemate thereof optionally in protected form and where neccesary deprotecting the compound thus obtained to form a compound of formula I or racemate thereof

c) for the preparation of a compound of formula I in which $R^3$ represents a moiety containing one or more free hydroxy groups or a free amino group, hydrolysing a corresponding compound in which $R^3$ represents a moiety containing one or more acyloxy or alkoxy groups or an acylamino group.

d) for the preparation of a compound of formula I in which $R^3$ represents a moiety which contains the $C_{1-6}$ alkanoyloxy group, the selective $C_{1-6}$ alkanoylation of a corresponding compound of the present invention in which $R^3$ represents a moiety which contains a hydroxy group

e) for the preparation of a compound of formula I in which X, $R^1$ and/or $R^3$ contains a $-SO_2$-moiety, oxidising a corresponding compound of the present invention in which X, $R^1$ and/or $R^3$ contains an -S- or $-SO_2$-moiety

f) for the preparation of a compound of formula I in which X, $R^1$ and/or $R^3$ contains a -SO- moiety, oxidising a corresponding compound of the present invention in which X, $R^1$ and/or $R^3$ contains an -S- moiety

g) for the preparation of a compound of formula I in which $R^1$ and/or $R^3$ contains a $C_{1-6}$ alkoxy, $C_{3-6}$ alkenyloxy or $C_{3-6}$ alkynyloxy group, alkylating, alkenylating or alkynylating a corresponding compound of the present invention in which $R^1$ and/or $R^3$ contains a hydroxy group

h) for the preparation of a compound of formula I in which $R^1$ and/or $R^3$ represents a moiety containing an acylamino group, acylating a corresponding compound of the present invention in which $R^1$ and/or $R^3$ represent a moiety containing an amine group and, whereafter, when the compound of formula I or a racemate thereof is obtained in the form of a base and a salt is required, reacting said compound of formula I obtained with an acid to form the salt.

**2.** A process as claimed in claim 1 wherein $R^1$ represents a phenyl or naphthyl group substituted by one or two substituents selected from halogen, trifluoromethyl, cyano, nitro, $C_{1-6}$alkyl or $C_{1-6}$alkoxy; or $R^1$ is a 5- or 6-membered heterocyclic ring containing one or two heteroatoms selected from oxygen, nitrogen or sulphur, the heterocyclic ring being fused with a benzene ring, and the heterocyclic ring and/or benzene ring being optionally substituted by halogen and/or hydroxy, linkage to the remainder of the compound of formula I being via the heterocyclic ring.

**3.** A process as claimed in claim 1 or 2 wherein $R^1$ represents a halophenyl, dihalophenyl, trifluoromethylphenyl, methoxyphenyl, methylphenyl, nitrophenyl, cyanophenyl, naphthyl, benzothienyl, benzofuranyl, benzoxazolyl, benzisoxazolyl optionally substituted by fluorine, benzimidazolyl optionally substituted by fluorine or 2-(1,3-dioxoisoindolinyl) group.

**4.** A process as claimed in any one of the preceding claims wherein X represents $-CH_2-$.

**5.** A process as claimed in any one of the preceding claims wherein $R^2$ represents methyl.

**6.** A process as claimed in any one of the preceding claims wherein $R^3$ represents an alkyl group having up to 7 carbon atoms optionally being substituted by hydroxy, $C_{1-6}$ alkylsulphide, $C_{1-6}$ alkylsulfinyl or $C_{1-6}$ alkoxy; or $R^3$ represents a phenyl group optionally substituted by one or two substituents selected from hydroxy, nitro, $C_{1-6}$ alkylsulphide, $C_{1-6}$ alkysulfinyl, $C_{1-6}$ alkylsulphonyl, $C_{1-6}$ alkoxy, amino, $C_{1-6}$ alkylamino or $C_{1-6}$ acylamino or $R^3$ represents a group of the formula $-D-R_6$ in which D represents a single bond or a $-CH(CH_3)$-group and $R_6$ represents a 5- or 6-membered heterocyclic ring containing one or two heteroatoms selected from oxygen, nitrogen or sulphur, or $R^3$ represents a benzyl group.

**7.** A process as claimed in any one of the preceding claims wherein $R^3$ represents an isopropyl, isobutyl, sec-butyl, t-butyl, 1-($C_{1-4}$alkoxy)ethyl, phenyl, hydroxyphenyl, 1-methylthioethyl, 1-morpholinoethyl, dimethoxyphenyl, hydroxymethylphenyl, aminophenyl, acetamidophenyl or methylaminophenyl group.

**8.** A process as claimed in claim 1 for the preparation of a compound of formula I selected from (2S)-N-[2-(N-methyl-3,4-dichlorophenylacetamido)-2-phenylethyl]pyrrolidine,
(2S)-N-[2-(N-methyl-4-trifluoromethylphenylacetamido)-2-phenylethyl]pyrrolidine,
(2S,3R)-N-[2-(N-methyl-3,4-dichlorophenylacetamido)-3-methoxybutyl]pyrrolidine, and
(2S)-N-[2-(N-methyl-3,4-dichlorophenylacetamido)-2-phenylethyl]$\Delta^3$-pyrroline and the salts thereof.

**9.** A process as claimed in any one of the preceding claims wherein the compound of formula (I) or racemate thereof is obtained in the form of a pharmaceutically acceptable salt thereof.

**Patentansprüche**
**Patentansprüche für folgende Vertragsstaaten : BE, CH, DE, FR, GB, IT, LI, NL**

**1.** Verbindung der Formel I,

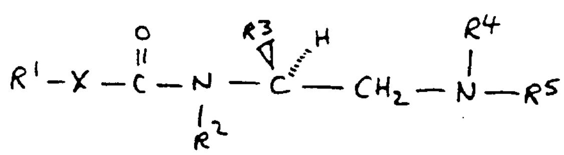

worin
$R^1$ für eine $C_{6-10}$-Aryl-Gruppe steht, die gegebenenfalls durch einen, zwei oder drei Substituenten substituiert ist, die unabhängig ausgewählt sind aus Halogen, Hydroxy, Trifluoromethyl, Cyano, Nitro, Amino, Aminocarbonyl, Carboxy, Sulfonsäure, ($C_{1-6}$-Alkoxy)carbonyl, $C_{1-6}$-Alkylsulfid, $C_{1-6}$-Alkylsulfoxid, $C_{1-6}$-Alkylsulfon, $C_{1-6}$-Alkanoyl, $C_{1-6}$-Alkoxy, $C_{3-6}$-Alkenyloxy, $C_{3-6}$-Alkinyloxy, $C_{1-6}$-Acylamino, $C_{1-6}$-Acylmethylamino, $C_{1-6}$-Alkyl, $C_{1-6}$-Monoalkylamino, ($C_{1-6}$-Monoalkylamino)carbonyl und Gruppen der Formel II,

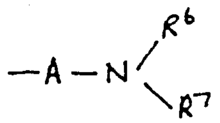

worin
A für -CO- oder eine einfache Bindung steht und $R^6$ und $R^7$, welche gleich oder verschieden sein können, jeweils für eine $C_{1-6}$-Alkyl-Gruppe stehen oder $R^6$ und $R^7$ zusammen mit dem sie verbindenden Stickstoffatom ein cyclisches Amin mit 4 bis 7 Ringatomen oder, sofern zweckmäßig, ein Oxyd davon bilden, oder
$R^1$ für eine heterocyclische Gruppierung mit einem 5- oder 6gliedrigen heterocyclischen Ring steht, der ein oder zwei Heteroatome enthält, die unabhängig ausgewählt sind aus Sauerstoff, Stickstoff und Schwefel, wobei der Ring gegebenenfalls mit einem Benzolring kondensiert ist, wobei weiterhin der heterocyclische und/oder Benzolring gegebenenfalls an Kohlenstoff durch einen oder mehrere Substituenten substituiert ist, die ausgewählt sind aus Amino-, Halogen, Hydroxy (und Ketotautomeren davon), $C_{1-6}$-Alkyl, $C_{1-6}$-Alkoxy, $C_{3-6}$-Alkenyloxy und $C_{3-6}$-Alkinyloxy, und wobei außerdem das Stickstoffheteroatom gegebenenfalls ein Sauerstoffatom oder eine Hydroxy- oder $C_{1-6}$-Alkyl-Gruppe trägt;
X für eine einfache Bindung, $-CH_2-$, $-OCH_2-$, $-SCH_2-$, $-SOCH_2-$, $-SO_2CH_2-$ oder $-CH_2-CH_2-$ steht;
$R^2$ für Wasserstoff oder $C_{1-3}$-Alkyl steht;
$R^3$ für eine Alkyl-, Cycloalkyl- oder Cycloalkylmethyl-Gruppe mit bis zu 7 Kohlenstoffatomen steht, wobei der Cycloalkyl-Teil, sofern anwesend, 3 bis 6 Kohlenstoffatome aufweist und wobei die genannte Gruppe gegebenenfalls durch einen oder mehrere Substituenten substituiert ist, die ausgewählt sind aus Hydroxy, Amino, Amidino, Guanidino, Aminocarbonyl, Carboxy, $C_{1-6}$-Alkoxy, ($C_{1-6}$-Alkoxy)-carbonyl, ($C_{3-6}$-Alkenyloxy)carbonyl, ($C_{3-6}$-Alkinyloxy)carbonyl, $C_{1-6}$-Alkanoyloxy, $C_{1-6}$-Alkylsulfid, $C_{1-6}$-Alkylsulfoxid, $C_{1-6}$-Alkylsulfon, $C_{1-6}$-(Monoalkylamino)carbonyl, $C_{1-6}$-Acylamino, $C_{1-6}$-Acylmethylamino, $C_{1-6}$-Monoalkylamino und den oben definierten Gruppen der Formel II, oder
$R^3$ für eine Gruppe $-B-R^1_a$ steht, worin B für $-CH_2-$, $-CH(CH_3)-$ oder eine einfache Bindung steht und $R^1_a$ für eine gegebenenfalls substituierte carbocyclische $C_{6-10}$-Aryl-Gruppe, wie sie oben für $R^1$ definiert ist, steht, oder
$R^3$ für eine Gruppe $-D-R_b$ steht, worin D für eine einfache Bindung $-CH_2-$, $-CH(CH_3)-$,$-CH_2-O-$, $-CH(CH_3)-O-$, $-CH_2-S-$, $-CH(CH_3)-S-$, $-CH_2-NH-$ oder$-CH(CH_3)-NH-$ steht und $R_b$ für einen 4- bis 6gliedrigen heterocyclischen Ring mit bis zu 4 Heteroatomen steht, die ausgewählt sind aus Sauerstoff, Schwefel und Stickstoff, wobei der heterocyclische Ring gegebenenfalls an Stickstoff oder Schwefel durch Sauerstoff oder an Stickstoff durch Hydroxy oder $C_{1-3}$-Alkyl substituiert ist und/oder wobei der Ring gegebenenfalls an Kohlenstoff durch einen oder mehrere Substituenten substituiert ist, die ausgewählt sind aus Amino, Hydroxy, Thio (und Tautomeren davon), Cyano, Halogen, $C_{1-3}$-Alkoxy, $C_{1-3}$-Monoalkylamino, $C_{1-3}$-Acylamino, $C_{1-3}$-Acylmethylamino, $C_{1-3}$-Alkylthio und Gruppen der oben definierten Formel II; und
$R^4$ und $R^5$, welche gleich oder verschieden sein können, jeweils für eine $C_{3-5}$-Alkenyl, $C_{3-5}$-Alkinyl, $C_{1-6}$-Alkyl oder $C_{4-7}$-Cycloalkylalkyl-Gruppe stehen oder
$R^4$ und $R^5$ zusammen mit dem dazwischenliegenden Stickstoffatom für einen 4- bis 7gliedrigen heterocyclischen Ring stehen, der gegebenenfalls ein weiteres Heteroatom enthält, das ausgewählt ist aus Sauerstoff und Schwefel;
sowie die Racemate davon und die Salze dieser Verbindungen und Racemate.

2. Verbindung nach Anspruch 1, worin $R^1$ für eine Phenyl- oder Naphthyl-Gruppe steht, die durch einen oder zwei Substituenten substituiert ist, welche ausgewählt sind aus Halogen, Trifluoromethyl, Cyano, Nitro, $C_{1-6}$-Alkyl und $C_{1-6}$-Alkoxy, oder

$R^1$ für einen 5- oder 6gliedrigen heterocyclischen Ring steht, der ein oder zwei Heteroatome enthält, die ausgewählt sind aus Sauerstoff, Stickstoff und Schwefel, wobei der heterocyclische Ring mit einem Benzol-Ring kondensiert ist, wobei weiterhin der heterocyclische Ring und/oder der Benzolring gegebenenfalls durch Halogen und/oder Hydroxy substituiert ist, und wobei außerdem die Verbindung mit dem Rest der Verbindung der Formel I über den heterocyclischen Ring erfolgt.

**3.** Verbindung nach Anspruch 1, worin $R^1$ für Halogenophenyl, Dihalogenophenyl, Trifluoromethylphenyl, Methoxyphenyl, Methylphenyl, Nitrophenyl, Cyanophenyl, Naphthyl, Benzothienyl, Benzofuranyl, Benzoxazolyl, Benzisoxazolyl, das gegebenenfalls durch Fluor substituiert ist, Benzimidazolyl, das gegebenenfalls durch Fluor substituiert ist, oder 2-(1,3-Dioxoisoindolinyl) steht.

**4.** Verbindung nach einem der Ansprüche 1 bis 3, worin $R^1$ für eine Halogenophenyl-, Dihalogenophenyl-, Nitrophenyl-, Cyanophenyl- oder Trifluoromethylphenyl-Gruppe steht.

**5.** Verbindung nach einem der vorhergehenden Ansprüche, worin $R^1$ für eine 3,4-Dichlorophenyl- oder 4-Trifluoromethylphenyl-Gruppe steht.

**6.** Verbindung nach einem der Ansprüche 1 bis 5, welche zwischen Octanol und einem wässerigen Puffer bei pH 7,4 einen Verteilungskoeffizienten von 1 oder mehr aufweist.

**7.** Verbindung nach einem der vorhergehenden Ansprüche, worin X für $-CH_2-$ steht.

**8.** Verbindung nach einem der vorhergehenden Ansprüche, worin $R^2$ für Methyl steht.

**9.** Verbindung nach einem der vorhergehenden Ansprüche, worin $R^3$ für eine Alkyl-Gruppe mit bis zu 7 Kohlenstoffatomen steht, die gegebenenfalls durch Hydroxy, $C_{1-6}$-Alkylsulfid, $C_{1-6}$-Alkylsulfinyl oder $C_{1-6}$-Alkoxy substituiert ist, oder
$R^3$ für eine Phenyl-Gruppe steht, die gegebenenfalls durch einen oder zwei Substituenten substituiert ist, die ausgewählt sind aus Hydroxy, Nitro, $C_{1-6}$-Alkylsulfid, $C_{1-6}$-Alkylsulfinyl, $C_{1-6}$-Alkylsulfonyl, $C_{1-6}$-Alkoxy, Amino, $C_{1-6}$-Alkylamino und $C_{1-6}$-Acylamino, oder
$R^3$ für eine Gruppe der Formel $-D-R_6$ steht, worin D für eine einfache Bindung oder eine Gruppe $-CH(CH_3)-$ steht und $R^6$ für einen 5- oder 6gliedrigen heterocyclischen Ring steht, der ein oder zwei Heteroatome enthält, die ausgewählt sind aus Sauerstoff, Stickstoff und Schwefel, oder
$R^3$ für eine Benzyl-Gruppe steht.

**10.** Verbindung nach einem der vorhergehenden Ansprüche, worin $R^3$ für eine Isopropyl-, Isobutyl-, sec-Butyl-, t-Butyl-, 1-($C_{1-4}$-Alkoxy)ethyl-, Phenyl-, Hydroxyphenyl-, 1-Methylthioethyl-, 1-Morpholinoethyl-, Dimethoxyphenyl-, Hydroxymethylphenyl-, Aminophenyl-, Acetamidophenyl- oder Methylaminophenyl-Gruppe steht.

**11.** Verbindung nach einem der vorhergehenden Ansprüche, worin $R^4$ und $R^5$ zusammen mit dem dazwischenliegenden Stickstoffatom für eine Pyrrolidino-, Pyperidino, $\Delta^3$-Pyrrolino-, Dimethylamino-, Diethylamino-, N-Allyl-N-methylamino- oder N-Isopropyl-N-methylamino-Gruppe stehen.

**12.** Verbindung nach einem der vorhergehenden Ansprüche, worin $R^4$ und $R^5$ zusammen mit dem dazwischenliegenden Stickstoffatom für eine Pyrrolidino-, $\Delta^3$-Pyrrolino- oder N-Isopropyl-N-methyla mino-Gruppe stehen.

**13.** Verbindung der Formel I, welche ausgewählt ist aus
(2S)-N-[2-(N-Methyl-3,4-dichlorophenylacetamido)-2-phenylethyl]pyrrolidin,
(2S)-N-[2-(N-Methyl-4-trifluoromethylphenylacetamido)-2-phenylethyl]pyrrolidin,
(2S,3R)-N-[2-(N-Methyl-3,4-dichlorophenylacetamido)-3-methoxybutyl]pyrrolidin und
(2S)-N-[2-(N-Methyl-3,4-dichlorophenylacetamido)-2-phenylethyl]$\Delta^3$-pyrrolin
sowie den Salzen davon.

**14.** Verbindungen nach einem der vorhergehenden Ansprüche, welche die Form eines pharmazeutisch zulässigen Salzes davon aufweisen.

**15.** Verfahren zur Herstellung einer Verbindung der Formel I von Anspruch 1 oder eines Salzes davon, bei welchem

a) eine Verbindung der Formel IV,

$R^1$ - X - Y

worin $R^1$ und X die oben angegebenen Bedeutungen besitzen und Y für eine Säuregruppe oder ein aktiviertes Derivat davon steht, mit einer Verbindung der Formel V,

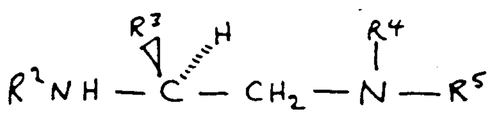

worin $R^2$, $R^3$, $R^4$ und $R^5$ die oben angegebenen Bedeutungen besitzen, oder ein Racemat davon, gegebenenfalls in einer geschützten Form, umgesetzt wird und die so erhaltene Verbindung nötigenfalls von Schutzgruppen befreit wird, um eine Verbindung der Formel I oder ein Racemat davon herzustellen;

b) zur Herstellung einer Verbindung der Formel I, worin $R^2$ für Wasserstoff steht, eine Verbindung der Formel III,

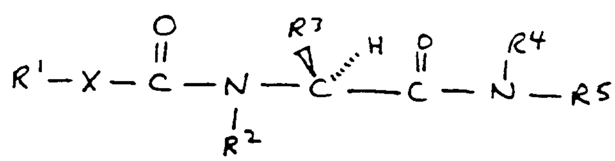

worin $R^1$, $R^3$, $R^4$, $R^5$ und X die oben angegebenen Bedeutungen besitzen, oder ein Racemat davon, gegebenenfalls in geschützter Form, selektiv reduziert wird und die so erhaltene Verbindung nötigenfalls von Schutzgruppen befreit wird, um eine Verbindung von I oder ein Racemat davon herzustellen;

c) zur Herstellung einer Verbindung der Formel I, worin $R^3$ für eine Gruppierung steht, die eine oder mehrere freie Hydroxy-Gruppen oder eine freie Amino-Gruppe enthält, eine entsprechende Verbindung, worin $R^3$ für eine Gruppierung steht, die eine oder mehrere Acyloxy- oder Alkoxy-Gruppen oder eine Acylamino-Gruppe enthält, hydrolysiert wird;

d) zur Herstellung einer Verbindung der Formel I , worin $R^3$ für eine Gruppierung steht, welche eine $C_{1-6}$-Alkanoyloxy-Gruppe enthält, eine entsprechende erfindungsgemäße Verbindung, worin $R^3$ für eine Gruppierung steht, die eine Hydroxy-Gruppe enthält, einer selectiven $C_{1-6}$-Alkanoylierung unterworfen wird;

e) zur Herstellung einer Verbindung der Formel I, worin X, $R^1$ und/oder $R^3$ eine Gruppierung $-SO_2-$ enthält, eine entsprechende erfindungsgemäße Verbindung, worin X, $R^1$ und/oder $R^3$ eine Gruppierung -S- oder -SO- enthält, oxidiert wird;

f) zur Herstellung einer Verbindung der Formel I, worin X, $R^1$ oder $R^3$ eine Gruppierung -SO- enthält, eine entsprechende erfindungsgemäße Verbindung, worin X, $R^1$ und/oder $R^3$ eine Gruppierung -S- enthält, oxidiert wird;

g) zur Herstellung einer Verbindung der Formel I, worin $R^1$ und/oder $R^3$ eine $C_{1-6}$-Alkoxy-, $C_{3-6}$-Alkenyloxy- oder $C_{3-6}$-Alkinyloxy-Gruppe enthält, eine entsprechende erfindungsgemäße Verbindung, worin $R^1$ und/oder $R^3$ eine Hydroxy-Gruppe enthält, alkyliert, alkenyliert oder alkinyliert wird;

h) zur Herstellung einer Verbindung der Formel I, worin $R^1$ und/oder $R^3$ für eine Gruppierung steht, die eine Acylamino-Gruppe enthält, eine entsprechende erfindungsgemäße Verbindung, worin $R^1$ und/oder $R^3$ für eine Gruppierung steht, die eine Amino-Gruppe enthält, acyliert wird;

worauf, wenn die Verbindung der Formel I oder das Racemat davon in Form einer Base erhalten wird und ein Salz gewünscht wird, die erhaltene Verbindung der Formel I zur Bildung eines Salzes mit einer Säure umgesetzt wird.

16. Pharmazeutische Zusammensetzung, welche als aktiven Bestandteil mindestens eine Verbindung der Formel I von Anspruch 1 oder ein Racemat davon oder ein pharmazeutisch zulässiges Salz dieser Verbindung oder dieses Racemats gemeinsam mit einem pharmazeutisch zulässigen Träger- oder Verdünnungsmittel enthält.

**Patentansprüche für folgenden Vertragsstaat : ES**

1. Verfahren zur Herstellung einer Verbindung der Formel I,

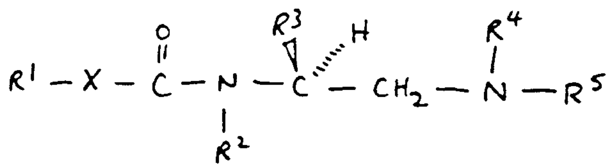

worin
$R^1$ für eine $C_{6-10}$-Aryl-Gruppe steht, die gegebenenfalls durch einen, zwei oder drei Substituenten substituiert ist, die unabhängig ausgewählt sind aus Halogen, Hydroxy, Trifluoromethyl, Cyano, Nitro, Amino, Aminocarbonyl, Carboxy, Sulfonsäure, ($C_{1-6}$-Alkoxy)carbonyl, $C_{1-6}$-Alkylsulfid, $C_{1-6}$-Alkylsulfoxid, $C_{1-6}$-Alkylsulfon, $C_{1-6}$-Alkanoyl, $C_{1-6}$-Alkoxy, $C_{3-6}$-Alkenyloxy, $C_{3-6}$-Alkinyloxy, $C_{1-6}$-Acylamino, $C_{1-6}$-Acylmethylamino, $C_{1-6}$-Alkyl, $C_{1-6}$-Monoalkylamino, ($C_{1-6}$-Monoalkylamino)carbonyl und Gruppen der Formel II,

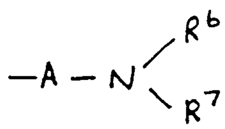

worin
A für -CO- oder eine einfache Bindung steht und $R^6$ und $R^7$, welche gleich oder verschieden sein können, jeweils für eine $C_{1-6}$-Alkyl-Gruppe stehen oder $R^6$ und $R^7$ zusammen mit dem sie verbindenden Stickstoffatom ein cyclisches Amin mit 4 bis 7 Ringatomen oder, sofern zweckmäßig, ein Oxyd davon bilden, oder
$R^1$ für eine heterocyclische Gruppierung mit einem 5- oder 6gliedrigen heterocyclischen Ring steht, der ein oder zwei Heteroatome enthält, die unabhängig ausgewählt sind aus Sauerstoff, Stickstoff und Schwefel, wobei der Ring gegebenenfalls mit einem Benzolring kondensiert ist, wobei weiterhin der heterocyclische und/oder Benzolring gegebenenfalls an Kohlenstoff durch einen oder mehrere Substituenten substituiert ist, die ausgewählt sind aus Amino-, Halogen, Hydroxy (und Ketotautomeren davon), $C_{1-6}$-Alkyl, $C_{1-6}$-Alkoxy, $C_{3-6}$-Alkenyloxy und $C_{3-6}$-Alkinyloxy, und wobei außerdem das Stickstoffheteroatom gegebenenfalls ein Sauerstoffatom oder eine Hydroxy- oder $C_{1-6}$-Alkyl-Gruppe trägt;
X für eine einfache Bindung, $-CH_2-$,$-OCH_2-$, $-SCH_2-$, $-SOCH_2-$, $-SO_2CH_2-$ oder $-CH_2-CH_2-$ steht;
$R^2$ für Wasserstoff oder $C_{1-3}$-Alkyl steht;
$R^3$ für eine Alkyl-, Cycloalkyl- oder Cycloalkylmethyl-Gruppe mit bis zu 7 Kohlenstoffatomen steht, wobei der Cycloalkyl-Teil, sofern anwesend, 3 bis 6 Kohlenstoffatome aufweist und wobei die genannte Gruppe gegebenenfalls durch einen oder mehrere Substituenten substituiert ist, die ausgewählt sind aus Hydroxy, Amino, Amidino, Guanidino, Aminocarbonyl, Carboxy, $C_{1-6}$-Alkoxy, ($C_{1-6}$-Alkoxy)-carbonyl, ($C_{3-6}$-Alkenyloxy)carbonyl, ($C_{3-6}$-Alkinyloxy)carbonyl, $C_{1-6}$-Alkanoyloxy, $C_{1-6}$-Alkylsufid, $C_{1-6}$-Akylsulfoxid, $C_{1-6}$-Alkylsulfon, $C_{1-6}$-(Monoalkylamino)carbonyl, $C_{1-6}$-Acylamino, $C_{1-6}$-Acylmethylamino, $C_{1-6}$-Monoalkylamino und den oben definierten Gruppen der Formel II, oder
$R^3$ für eine Gruppe $-B-R^1_a$ steht, worin B für $-CH_2-$, $-CH(CH_3)-$ oder eine einfache Bindung steht und $R^1_a$ für eine gegebenenfalls substituierte carbocyclische $C_{6-10}$-Aryl-Gruppe, wie sie oben für $R^1$ definiert ist, steht, oder
$R^3$ für eine Gruppe $-D-R_b$ steht, worin D für eine einfache Bindung $-CH_2-$, $-CH(CH_3)-$,$-CH_2-O-$, $-CH(CH_3)-O-$, $-CH_2-S-$, $-CH(CH_3)-S-$, $-CH_2-NH-$ oder $-CH(CH_3)-NH-$ steht und $R_b$ für einen 4 bis 6gliedrigen heterocyclischen Ring mit bis zu 4 Heteroatomen steht, die ausgewählt sind aus Sauerstoff, Schwefel

und Stickstoff, wobei der heterocyclische Ring gegebenenfalls an Stickstoff oder Schwefel durch Sauerstoff oder an Stickstoff durch Hydroxy oder $C_{1-3}$-Alkyl substituiert ist und/oder wobei der Ring gegebenenfalls an Kohlenstoff durch einen oder mehrere Substituenten substituiert ist, die ausgewählt sind aus Amino, Hydroxy, Thio (und Tautomeren davon), Cyano, Halogen, $C_{1-3}$-Alkoxy, $C_{1-3}$-Monoalkylamino, $C_{1-3}$-Acylamino, $C_{1-3}$-Acylmethylamino, $C_{1-3}$-Alkylthio und Gruppen der oben definierten Formel II, und

$R^4$ und $R^5$, welche gleich oder verschieden sein können, jeweils für eine $C_{3-5}$-Alkenyl, $C_{3-5}$-Alkinyl, $C_{1-6}$-Alkyl oder $C_{4-7}$-Cycloalkylalkyl-Gruppe stehen oder

$R^4$ und $R^5$ zusammen mit dem dazwischenliegenden Stickstoffatom für einen 4- bis 7gliedrigen heterocyclischen Ring stehen, der gegebenenfalls ein weiteres Heteroatom enthält, das ausgewählt ist aus Sauerstoff und Schwefel;

oder eines Racemats davon oder eines Salzes dieser Verbindung oder dieses Racemats, bei welchem

a) eine Verbindung der Formel IV,

$R^1$ - X - Y

worin $R^1$ und X die oben angegebenen Bedeutungen besitzen und Y für eine Säuregruppe oder ein aktiviertes Derivat davon steht, mit einer Verbindung der Formel V, worin $R^2$, $R^3$, $R^4$ und $R^5$ die oben angegebenen Bedeutungen besitzen, oder ein Racemat davon, gegebenenfalls in einer geschützten Form, umgesetzt wird und die so erhaltene Verbindung nötigenfalls von Schutzgruppen befreit wird, um eine Verbindung der Formel I oder ein Racemat davon herzustellen;

b) zur Herstellung einer Verbindung der Formel I, worin $R^2$ für Wasserstoff steht, eine Verbindung der Formel III,

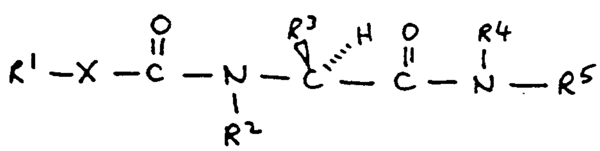

worin $R^1$, $R^3$, $R^4$, $R^5$ und X die oben angegebenen Bedeutungen besitzen, oder ein Racemat davon, gegebenenfalls in geschützter Form, selektiv reduziert wird und die so erhaltene Verbindung nötigenfalls von Schutzgruppen befreit wird, um eine Verbindung der Formel I oder ein Racemat davon herzustellen;

c) zur Herstellung einer Verbindung der Formel I, worin $R^3$ für eine Gruppierung steht, die eine oder mehrere freie Hydroxy-Gruppen oder eine freie Amino-Gruppe enthält, eine entsprechende Verbindung, worin $R^3$ für eine Gruppierung steht, die eine oder mehrere Acyloxy- oder Alkoxy-Gruppen oder eine Acylamino-Gruppe enthält, hydrolysiert wird;

d) zur Herstellung einer Verbindung der Formel I, worin $R^3$ für eine Gruppierung steht, welche eine $C_{1-6}$-Alkanoyloxy-Gruppe enthält, eine entsprechende erfindungsgemäße Verbindung, worin $R^3$ für eine Gruppierung steht, die eine Hydroxy-Gruppe enthält, einer selektiven $C_{1-6}$-Alkanoylierung unterworfen wird;

e) zur Herstellung einer Verbindung der Formel I, worin X, $R^1$ und/oder $R^3$ eine Gruppierung -$SO_2$- enthält, eine entsprechende erfindungsgemäße Verbindung, worin X, $R^1$ und/oder $R^3$ eine Gruppierung -S- oder -SO- enthält, oxidiert wird;

f) zur Herstellung einer Verbindung der Formel I, worin X, $R^1$ oder $R^3$ eine Gruppierung -SO- enthält, eine entsprechende erfindungsgemäße Verbindung, worin X, $R^1$ und/oder $R^3$ eine Gruppierung -S- enthält, oxidiert wird;

g) zur Herstellung einer Verbindung der Formel I, worin $R^1$ und/oder $R^3$ eine $C_{1-6}$-Alkoxy-, $C_{3-6}$-Alkenyloxy- oder oder $C_{3-6}$-Alkinyloxy-Gruppe enthält, eine entsprechende erfindungsgemäße Verbindung, worin $R^1$ und/oder $R^3$ eine Hydroxy-Gruppe enthält, alkyliert, alkenyliert oder alkinyliert wird;

h) zur Herstellung einer Verbindung der Formel I, worin $R^1$ und/oder $R^3$ für eine Gruppierung steht, die eine Acylamino-Gruppe enthält, eine entsprechende erfindungsgemäße Verbindung, worin $R^1$ und/oder $R^3$ für eine Gruppierung steht, die eine Amino-Gruppe enthält, acyliert wird;

worauf, wenn die Verbindung der Formel I oder das Racemat davon in Form einer Base erhalten wird

und ein Salz gewünscht wird, die erhaltene Verbindung der Formel I zur Bildung eines Salzes mit einer Säure umgesetzt wird.

2. Verfahren nach Anspruch 1, bei welchem $R^1$ für eine Phenyl- oder Naphthyl-Gruppe steht, die durch einen oder zwei Substituenten substituiert ist, welche ausgewählt sind aus Halogen, Trifluoromethyl, Cyano, Nitro, $C_{1-6}$-Alkyl und $C_{1-6}$-Alkoxy, oder $R^1$ für einen 5- oder 6gliedrigen heterocyclischen Ring steht, der ein oder zwei Heteroatome enthält, die ausgewählt sind aus Sauerstoff, Stickstoff und Schwefel, wobei der heterocyclische Ring mit einem Benzol-Ring kondensiert ist, wobei weiterhin der heterocyclische Ring und/oder der Benzolring gegebenenfalls durch Halogen und/oder Hydroxy substituiert ist, und wobei außerdem die Verbindung mit dem Rest der Verbindung der Formel I über den heterocyclischen Ring erfolgt.

3. Verfahren nach Anspruch 1 oder 2, bei welchem $R^1$ für Halogenophenyl, Dihalogenophenyl, Trifluoromethylphenyl, Methoxyphenyl, Methylphenyl, Nitrophenyl, Cyanophenyl, Naphthyl, Benzothienyl, Benzofuranyl, Benzoxazolyl, Benzisoxazolyl, das gegebenenfalls durch Fluor substituiert ist, Benzimidazolyl, das gegebenenfalls durch Fluor substituiert ist, oder 2-(1,3-Dioxoisoindolinyl) steht.

4. Verfahren nach einem der vorhergehenden Ansprüche, bei welchem X für $-CH_2-$ steht.

5. Verfahren nach einem der vorhergehenden Ansprüche, bei welchem $R^2$ für Methyl steht.

6. Verfahren nach einem der vorhergehenden Ansprüche, bei welchem $R^3$ für eine Alkyl-Gruppe mit bis zu 7 Kohlenstoffatomen steht, die gegebenenfalls durch Hydroxy, $C_{1-6}$-Alkylsulfid, $C_{1-6}$-Alkylsulfinyl oder $C_{1-6}$-Alkoxy substituiert ist, oder
$R^3$ für eine Phenyl-Gruppe steht, die gegebenenfalls durch einen oder zwei Substituenten substituiert ist, die ausgewählt sind aus Hydroxy, Nitro, $C_{1-6}$-Alkylsulfid, $C_{1-6}$- Alkylsulfinyl, $C_{1-6}$-Alkylsulfonyl, $C_{1-6}$-Alkoxy, Amino, $C_{1-6}$- Alkylamino und $C_{1-6}$-Acylamino, oder
$R^3$ für eine Gruppe der Formel $-D-R_6$ steht, worin D für eine einfache Bindung oder eine Gruppe $-CH(CH_3)-$ steht und $R^6$ für einen 5- oder 6gliedrigen heterocyclischen Ring steht, der ein oder zwei Heteroatome enthält, die ausgewählt sind aus Sauerstoff, Stickstoff und Schwefel, oder
$R^3$ für eine Benzyl-Gruppe steht.

7. Verfahren nach einem der vorhergehenden Ansprüche, bei welchem $R^3$ für eine Isopropyl-, Isobutyl-, sec-Butyl-, t-Butyl-, 1-($C_{1-4}$-Alkoxy)ethyl-, Phenyl-, Hydroxyphenyl-, 1-Methylthioethyl-, 1-Morpholinoethyl-, Dimethoxyphenyl-, Hydroxymethylphenyl-, Aminophenyl-, Acetamidophenyl- oder Methylaminophenyl-Gruppe steht.

8. Verfahren nach Anspruch 1 zur Herstellung einer Verbindung der Formel I, welche ausgewählt ist aus
(2S)-N-[2-(N-Methyl-3,4-dichlorophenylacetamido)-2-phenylethyl]pyrrolidin,
(2S)-N-[2-(N-Methyl-4-trifluoromethylphenylacetamido)-2-phenylethyl]pyrrolidin,
(2S,3R)-N-[2-(N-Methyl-3,4-dichlorophenylacetamido)-3-methoxybutyl]pyrrolidin und
(2S)-N-[2-(N-Methyl-3,4-dichlorophenylacetamido)-2-phenylethyl]$\Delta^3$-pyrrolin
sowie den Salzen davon.

9. Verfahren nach einem der vorhergehenden Ansprüche, bei welchem die Verbindung der Formel I oder das Racemat davon in Form eines pharmazeutisch zulässigen Salzes davon erhalten wird.

**Revendications**
**Revendications pour les Etats contractants suivants : BE, CH, DE, FR, GB, IT, LI, NL**

1. Composé de formule I

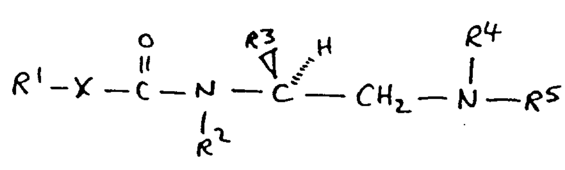

[dans laquelle $R^1$ représente un groupe aryle en $C_6$ à $C_{10}$, facultativement substitué par un, deux ou trois substituants choisis indépendamment entre un halogène, un groupe hydroxy, trifluorométhyle, cyano, nitro, amino, aminocarbonyle, carboxy, acide sulfonique, (alkoxy en $C_1$ à $C_6$)-carbonyle,alkylsulfure en $C_1$ à $C_6$, alkylsulfoxyde en $C_1$ à $C_6$, alkylsulfone en $C_1$ à $C_6$, alcanoyle en $C_1$ à $C_6$, alkoxy en $C_1$ à $C_6$, alcényloxy en $C_3$ à $C_6$, alcynyloxy en $C_3$ à $C_6$, acylamino en $C_1$ à $C_6$, (acyle en $C_1$ à $C_6$)-méthylamino, alkyle en $C_1$ à $C_6$, monoalkylamino en $C_1$ à $C_6$, (monoalkylamino en $C_1$ à $C_6$)carbonyle et un groupe de formule II

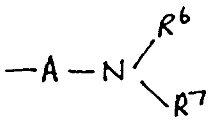

dans laquelle A est un groupe -CO- ou une liaison simple et $R^6$ et $R^7$, qui peuvent être identiques ou différents, représentent chacun un groupe alkyle en $C_1$ à $C_6$, ou bien $R^6$ et $R^7$ forment, conjointement avec l'atome intermédiaire d'azote, une amine cyclique à noyau de 4 à 7 atomes et, dans les cas appropriés, les oxydes correspondants, ou bien $R^1$ représente un groupement hétérocyclique comprenant un noyau hétérocyclique pentagonal ou hexagonal qui contient un ou deux hétéroatomes choisis indépendamment entre des atomes d'oxygène, d'azote et de soufre, le noyau étant facultativement condensé à un noyau benzénique et le noyau hétérocylique et/ou benzénique étant facultativement substitué sur du carbone par un ou plusieurs substituants choisis entre un groupe amino, un halogène, un groupe hydroxy (et ses formes céto-tautomères), un groupe alkyle en $C_1$ à $C_6$, un groupe alkoxy en $C_1$ à $C_6$, un groupe alcényloxy en $C_3$ à $C_6$ et un groupe alcynyloxy en $C_3$ à $C_6$, tout hétéroatome d'azote portant facultativement un atome d'oxygène ou un groupe hydroxy ou alkyle en $C_1$ à $C_3$ ;

X représente une liaison simple, un groupe $-CH_2-$, $-OCH_2-$, $-SCH_2-$, $-SOCH_2-$, $-SO_2CH_2-$ ou $-CH_2-CH_2-$ ;

$R^2$ représente l'hydrogène ou un groupe alkyle en $C_1$ à $C_3$ ;

$R^3$ représente un groupe alkyle, cycloalkyle ou cycloalkylméthyle ayant jusqu'à 7 atomes de carbone, la portion cycloalkyle, lorsqu'elle est présente, ayant 3 à 6 atomes de carbone, ce groupe étant facultativement substitué par un ou plusieurs substituants choisis entre un groupe hydroxy, amino, amidino, guanidino, aminocarbonyle, carboxy, alkoxy en $C_1$ à $C_6$, (alkoxy en $C_1$ à $C_6$)carbonyle, (alcényloxy en $C_3$ à $C_6$)carbonyle, (alcynyloxy en $C_3$ à $C_6$)carbonyle, alcanoyloxy en $C_1$ à $C_6$, alkylsulfure en $C_1$ à $C_6$, alkylsulfoxyde en $C_1$ à $C_6$, alkylsulfone en $C_1$ à $C_6$, (monoalkylamino en $C_1$ à $C_6$)carbonyle, acylamino en $C_1$ à $C_6$, (acyle en $C_1$ à $C_6$)méthylamino, monoalkylamino en $C_1$ à $C_6$, un groupe de formule II ;

ou bien $R^3$ représente le groupe $-B-R^1_a$ dans lequel B est un groupe $-CH_2-$, $-CH(CH_3)-$ ou une liaison simple, et $R^1_a$ représente un groupe aryle carbocyclique en $C_6$ à $C_{10}$ éventuellement substitué tel que défini pour $R^1$ ; ou bien

$R^3$ représente le groupe $-D-R_b$ dans lequel D est une liaison simple, un groupe $-CH_2-$, $-CH(CH_3)-$, $-CH_2-O-$, $-CH(CH_3)-O-$, $-CH_2-S-$, $-CH(CH_3)-S-$, $-CH_2-NH-$ ou $-CH(CH_3)-NH-$ et $R_b$ représente un noyau hétérocyclique tétragonal à hexagonal contenant jusqu'à 4 hétéroatomes choisis entre les atomes d'oxygène, de soufre et d'azote, le noyau hétérocyclique étant facultativement substitué sur l'azote ou le soufre par de l'oxygène ou sur l'azote par un groupe hydroxy ou un groupe alkyle en $C_1$ à $C_3$, et/ou le noyau étant facultativement substitué sur du carbone par un ou plusieurs substituants choisis entre des substituants amino, hydroxy, thio (et leurs tautomères), cyano, halogéno, alkoxy en $C_1$ à $C_3$, monoalkylamino en $C_1$ à $C_3$, (acyle en $C_1$ à $C_3$)amino, (acyle en $C_1$ à $C_3$)méthylamino, alkylthio en $C_1$ à $C_3$ et le groupe de formule II tel que précédemment défini,

et $R^4$ et $R^5$, qui peuvent être identiques ou différents, représentent chacun un groupe alcényle en $C_3$ à $C_5$, alcynyle en $C_3$ à $C_5$, alkyle en $C_1$ à $C_6$ ou cycloalkylalkyle en $C_4$ à $C_7$ ;

ou bien $R^4$ et $R^5$ représentent, avec l'atome intermédiaire d'azote, un noyau hétérocyclique tétragonal à heptagonal qui contient facultativement un autre hétéroatome choisi entre oxygène et soufre] ou un racémate de ce composé et les sels de ce composé ou de ce racémate.

2. Composé suivant la revendication 1, dans lequel $R^1$ représente un groupe phényle ou naphtyle substitué par un ou deux substituants choisis entre des substituants halogéno, trifluorométhyle, cyano, nitro, alkyle en $C_1$ à $C_6$ ou alkoxy en $C_1$ à $C_6$ ; ou bien $R^1$ est un noyau hétérocyclique pentagonal ou hexagonal contenant un ou deux hétéroatomes choisis entre oxygène, azote ou soufre, le noyau

hétérocyclique étant condensé avec un noyau benzénique, et le noyau hétérocyclique et/ou le noyau benzénique étant facultativement substitués par un halogène et/ou un radical hydroxy, la liaison avec le reste du composé de formule I s'effectuant par l'intermédiaire du noyau hétérocyclique.

**3.** Composé suivant la revendication 1, dans lequel $R^1$ représente un groupe halogénophényle, dihalogénophényle, trifluorométhylphényle, méthoxyphényle, méthylphényle, nitrophényle, cyanophényle, naphtyle, benzothiényle, benzofurannyle, benzoxazolyle, benzisoxazolyle, facultativement substitué par du fluor, benzimidazolyle facultativement substitué par du fluor ou un groupe 2-(1,3-dioxoisoindolinyle).

**4.** Composé suivant l'une quelconque des revendications 1 à 3, dans lequel $R^1$ représente un groupe halogénophényle, dihalogénophényle, nitrophényle, cyanophényle ou trifluorométhylphényle.

**5.** Composé suivant l'une quelconque des revendications précédentes, dans lequel $R^1$ représente un groupe 3,4-dichlorophényle ou 4-trifluorométhylphényle.

**6.** Composé suivant l'une quelconque des revendications 1 à 5, ayant un coefficient de distribution entre l'octanol et un tampon aqueux égal ou supérieur à 1, à un pH de 7,4.

**7.** Composé suivant l'une quelconque des revendications précédentes, dans lequel X représente $-CH_2-$.

**8.** Composé suivant l'une quelconque des revendications précédentes, dans lequel $R^2$ représente le groupe méthyle.

**9.** Composé suivant l'une quelconque des revendications précédentes, dans lequel $R^3$ représente un groupe alkyle ayant jusqu'à 7 atomes de carbone, facultativement substitué par un radical hydroxy, alkylsulfure en $C_1$ à $C_6$, alkylsulfinyle en $C_1$ à $C_6$ ou alkoxy en $C_1$ à $C_6$ ; ou bien $R^3$ représente un groupe phényle facultativement substitué par un ou deux substituants choisis entre des groupes hydroxy, nitro, alkylsulfure en $C_1$ à $C_6$, alkylsulfinyle en $C_1$ à $C_6$, alkylsulfonyle en $C_1$ à $C_6$, alkoxy en $C_1$ à $C_6$, amino, alkylamino en $C_1$ à $C_6$ ou acylamino en $C_1$ à $C_6$, ou bien $R^3$ représente un groupe de formule $-D-R_6$ dans laquelle D représente une liaison simple ou un groupe $-CH(CH_3)-$ et $R_6$ représente un noyau hétérocyclique pentagonal ou hexagonal contenant un ou deux hétéroatomes choisis entre oxygène, azote ou soufre, ou bien $R^3$ représente un groupe benzyle.

**10.** Composé suivant l'une quelconque des revendications précédentes, dans lequel $R^3$ représente un groupe isopropyle, isobutyle, sec.-butyle, tertio-butyle, 1-(alkoxy en $C_1$ à $C_4$)éthyle, phényle, hydroxyphényle, 1-méthylthioéthyle, 1-morpholinoéthyle, diméthoxyphényle, hydroxyméthylphényle, aminophényle, acétamidophényle ou méthylaminophényle.

**11.** Composé suivant l'une quelconque des revendications précédentes, dans lequel $R^4$ et $R^5$ représentent conjointement avec l'atome intermédiaire d'azote un groupe pyrrolidino, pipéridino, $\Delta^3$-pyrrolino, diméthylamino, diéthylamino, N-allyl-N-méthylamino ou N-isopropyl-N-méthylamino.

**12.** Composé suivant l'une quelconque des revendications prédédentes, dans lequel $R^4$ et $R^5$ représentent conjointement avec l'atome intermédiaire d'azote un groupe pyrrolidino, $\Delta^3$-pyrrolino ou N-isopropyl-N-méthylamino.

**13.** Composé de formule I, choisi entre
la (2S)-N-[2-(N-méthyl-3,4-dichlorophénylacétamido)-2-phényléthyl]pyrrolidine.
la (2S)-N-[2-(N-méthyl-4-trifluorométhylphénylacétamido)-2-phényléthyl]pyrrolidine,
la (2S,3R)-N-[2-(N-méthyl-3,4-dichlorophénylacétamido)-3-méthoxybutyl]pyrrolidine, et
la (2S)-N-[2-(N-méthyl-3,4-dichlorophénylacétamido)-2-[phényléthyl]$\Delta^3$-pyrroline et leurs sels.

**14.** Composé suivant l'une quelconque des revendications précédentes, sous la forme d'un sel acceptable du point de vue pharmaceutique.

**15.** Procédé de production d'un composé de formule I tel que défini dans la revendication 1 ou d'un sel de ce composé, qui comprend :

a) la réaction d'un composé de formule IV

$R^1$-X-Y

(dans laquelle $R^1$ et X sont tels que définis ci-dessus et Y représente un acide ou un dérivé activé de cet acide) avec un composé de formule V

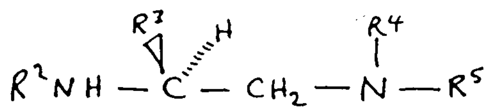

(dans laquelle $R^2$, $R^3$, $R^4$ et $R^5$ sont tels que définis ci-dessus) ou un racémate de ce composé facultativement sous une forme protégée, et si nécessaire, l'élimination de la protection du composé ainsi obtenu pour former un composé de formule I ou un racémate de ce composé

b) pour l'obtention d'un composé de formule I dans laquelle $R^2$ est l'hydrogène, la réduction sélective d'un composé de formule III

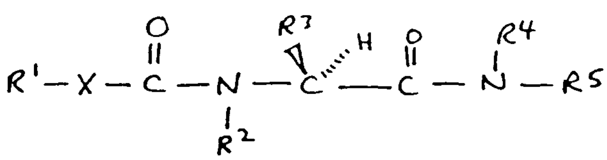

dans laquelle $R^1$, $R^3$, $R^4$, $R^5$ et X sont tels que définis ci-dessus, ou d'un racémate de ce composé facultativement sous une forme protégée et, si nécessaire, l'élimination de la protection du composé ainsi obtenu pour former un composé de formule I ou un racémate de ce composé

c) pour l'obtention d'un composé de formule I dans laquelle $R^3$ représente un groupement contenant un ou plusieurs groupes hydroxy libres ou un groupe amino libre, l'hydrolyse d'un composé correspondant dans lequel $R^3$ représente un groupement contenant un ou plusieurs groupes acyloxy ou alkoxy ou un groupe acylamino

d) pour l'obtention d'un composé de formule I dans laquelle $R^3$ représente un groupement qui contient le groupe alcanoyloxy en $C_1$ à $C_6$, l'alcanoylation en $C_1$ à $C_6$ sélective d'un composé correspondant de la présente invention dans lequel $R^3$ représente un groupement qui contient un groupe hydroxy

e) pour l'obtention d'un composé de formule I dans laquelle X, $R^1$ et/ou $R^3$ contiennent un groupe $-SO_2$, l'oxydation d'un composé correspondant de la présente invention dans lequel X, $R^1$ et/ou $R^3$ contiennent un groupe -S- ou $-SO_2$-

f) pour l'obtention d'un composé de formule I dans laquelle X, $R^1$ et/ou $R^3$ contiennent un groupe -SO-, l'oxydation d'un composé correspondant de la présente invention dans lequel X, $R^1$ et/ou $R^3$ contiennent un groupe -S- et

g) pour l'obtention d'un composé de formule I dans laquelle $R^1$ et/ou $R^3$ contiennent un groupe alkoxy en $C_1$ à $C_6$, alcényloxy en $C_3$ à $C_6$ ou alcynyloxy en $C_3$ à $C_6$, l'alkylation, l'alcénylation ou l'alcynylation d'un composé correspondant de la présente invention dans lequel $R^1$ et/ou $R^3$ contiennent un groupe hydroxy

h) pour l'obtention d'un composé de formule I dans laquelle $R^1$ et/ou $R^3$ représentent un groupement contenant un groupe acylamino, l'acylation d'un composé correspondant de la présente invention dans lequel $R^1$ et/ou $R^3$ représentent un groupement contenant un groupe amino

après quoi, lorsque le composé de formule I ou un racémate de ce composé est obtenu sous la forme d'une base et que l'on désire un sel, le composé de formule I obtenu est amené à réagir avec un acide pour former le sel.

**16.** Composition pharmaceutique comprenant comme ingrédient actif au moins un composé de formule I suivant la revendication 1 ou un racémate de ce composé ou un sel acceptable du point de vue pharmaceutique dudit composé ou du racémate en association avec un support ou diluant acceptable

du point de vue pharmaceutique.

**Revendications pour l'Etat contractant suivant : ES**

**1.** Procédé de production d'un composé de formule I

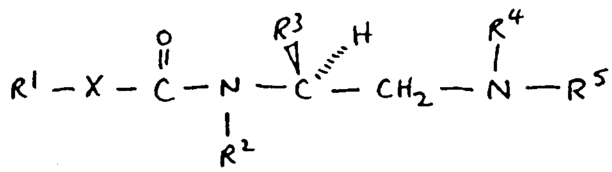

[dans laquelle $R^1$ représente un groupe aryle en $C_6$ à $C_{10}$, facultativement substitué par un, deux ou trois substituants choisis indépendamment entre un halogène, un groupe hydroxy, trifluorométhyle, cyano, nitro, amino, aminocarbonyle, carboxy, acide sulfonique, (alkoxy en $C_1$ à $C_6$)-carbonyle,alkylsulfure en $C_1$ à $C_6$, alkylsulfoxyde en $C_1$ à $C_6$, alkylsulfone en $C_1$ à $C_6$, alcanoyle en $C_1$ à $C_6$, alkoxy en $C_1$ à $C_6$, alcényloxy en $C_3$ à $C_6$, alcynyloxy en $C_3$ à $C_6$, acylamino en $C_1$ à $C_6$, (acyle en $C_1$ à $C_6$)-méthylamino, alkyle en $C_1$ à $C_6$, monoalkylamino en $C_1$ à $C_6$, (monoalkylamino en $C_1$ à $C_6$)carbonyle et un groupe de formule II

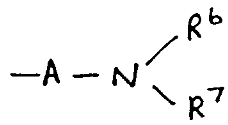

dans laquelle A est un groupe -CO- ou une liaison simple et $R^6$ et $R^7$, qui peuvent être identiques ou différents, représentent chacun un groupe alkyle en $C_1$ à $C_6$, ou bien $R^6$ et $R^7$ forment, conjointement avec l'atome intermédiaire d'azote, une amine cyclique à noyau de 4 à 7 atomes et, dans les cas appropriés, les oxydes correspondants, ou bien $R^1$ représente un groupement hétérocyclique comprenant un noyau hétérocyclique pentagonal ou hexagonal qui contient un ou deux hétéroatomes choisis indépendamment entre des atomes d'oxygène, d'azote et de soufre, le noyau étant facultativement condensé à un noyau benzénique et le noyau hétérocylique et/ou benzénique étant facultativement substitué sur du carbone par un ou plusieurs substituants choisis entre un groupe amino, un halogène, un groupe hydroxy (et ses céto-tautomères), un groupe alkyle en $C_1$ à $C_6$, un groupe alkoxy en $C_1$ à $C_6$, un groupe alcényloxy en $C_3$ à $C_6$ et un groupe alcynyloxy en $C_3$ à $C_6$, tout hétéroatome d'azote portant facultativement un atome d'oxygène ou un groupe hydroxy ou alkyle en $C_1$ à $C_3$ ;

X représente une liaison simple, un groupe $-CH_2-$, $-OCH_2-$, $-SCH_2-$, $-SOCH_2-$, $-SO_2CH_2-$ ou $-CH_2-CH_2-$ ;

$R^2$ représente l'hydrogène ou un groupe alkyle en $C_1$ à $C_3$ ;

$R^3$ représente un groupe alkyle, cycloalkyle ou cycloalkylméthyle ayant jusqu'à 7 atomes de carbone, la portion cycloalkyle, lorsqu'elle est présente, ayant 3 à 6 atomes de carbone, ce groupe étant facultativement substitué par un ou plusieurs substituants choisis entre un groupe hydroxy, amino, amidino, guanidino, aminocarbonyle, carboxy, alkoxy en $C_1$ à $C_6$, (alkoxy en $C_1$ à $C_6$)carbonyle, (alcényloxy en $C_3$ à $C_6$)carbonyle, (alcynyloxy en $C_3$ à $C_6$)carbonyle, alcanoyloxy en $C_1$ à $C_6$, alkylsulfure en $C_1$ à $C_6$, alkylsulfoxyde en $C_1$ à $C_6$, alkylsulfone en $C_1$ à $C_6$, (monoalkylamino en $C_1$ à $C_6$)carbonyle, acylamino en $C_1$ à $C_6$, (acyle en $C_1$ à $C_6$)méthylamino, monoalkylamino en $C_1$ à $C_6$, un groupe de formule II (comme défini ci-dessus) ;

ou bien $R^3$ représente le groupe $-B-R^1_a$ dans lequel B est un groupe $-CH_2-$, $-CH(CH_3)-$ ou une liaison simple et $R^1_a$ représente un groupe aryle carbocyclique en $C_6$ à $C_{10}$ éventuellement substitué tel que défini pour $R^1$ ; ou bien

$R^3$ représente le groupe $-D-R_b$ dans lequel D est une liaison simple, un groupe $-CH_2-$, $-CH(CH_3)-$, $-CH_2-O-$, $-CH(CH_3)-O-$, $-CH_2-S-$, $-CH(CH_3)-S-$, $-CH_2-NH-$ ou $-CH(CH_3)-NH-$ et $R_b$ représente un noyau hétérocyclique tétragonal à hexagonal contenant jusqu'à 4 hétéroatomes choisis entre les atomes d'oxygène, de soufre et d'azote, le noyau hétérocyclique étant facultativement substitué sur l'azote ou le soufre par de l'oxygène ou sur l'azote par un groupe hydroxy ou un groupe alkyle en $C_1$ à $C_3$, et/ou

le noyau étant facultativement substitué sur du carbone par un ou plusieurs substituants choisis entre des substituants amino, hydroxy, thio (et leurs tautomères), cyano, halogéno, alkoxy en $C_1$ à $C_3$, monoalkylamino en $C_1$ à $C_3$, (acyle en $C_1$ à $C_3$)amino, (acyle en $C_1$ à $C_3$)méthylamino, alkylthio en $C_1$ à $C_3$ et le groupe de formule II tel que précédemment défini,

et $R^4$ et $R^5$, qui peuvent être identiques ou différents, représentent chacun un groupe alcényle en $C_3$ à $C_5$, alcynyle en $C_3$ à $C_5$, alkyle en $C_1$ à $C_6$ ou cycloalkylalkyle en $C_4$ à $C_7$ ;

ou bien $R^4$ et $R^5$ représentent, avec l'atome intermédiaire d'azote, un noyau hétérocyclique tétragonal à heptagonal qui contient facultativement un autre hétéroatome choisi entre oxygène et soufre] ou d'un racémate de ce composé et des sels de ce composé ou de ce racémate, qui comprend :

a) la réaction d'un composé de formule IV

$R^1$-X-Y

(dans laquelle $R^1$ et X sont tels que définis ci-dessus et Y représente un acide ou un dérivé activé d'un acide) avec un composé de formule V

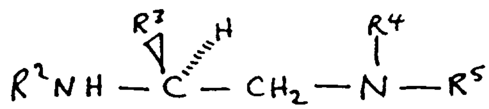

(dans laquelle $R^2$, $R^3$, $R^4$ et $R^5$ sont tels que définis ci-dessus) ou un racémate de ce composé facultativement sous une forme protégée, et si nécessaire, l'élimination de la protection du composé ainsi obtenu pour former un composé de formule I ou un racémate de ce composé

b) pour l'obtention d'un composé de formule I dans laquelle $R^2$ est l'hydrogène, la réduction sélective d'un composé de formule III,

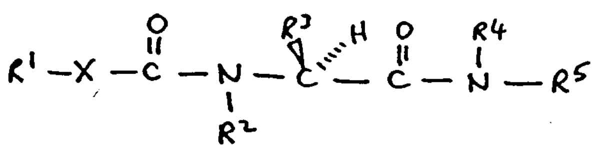

dans laquelle $R^1$, $R^3$, $R^4$, $R^5$ et X sont tels que définis ci-dessus, ou d'un racémate de ce composé facultativement sous une forme protégée et, si nécessaire, l'élimination de la protection du composé ainsi obtenu pour former un composé de formule I ou un racémate de ce composé

c) pour l'obtention d'un composé de formule I dans laquelle $R^3$ représente un groupement contenant un ou plusieurs groupes hydroxy libres ou un groupe amino libre, l'hydrolyse d'un composé correspondant dans lequel $R^3$ représente un groupement contenant un ou plusieurs groupes acyloxy ou alkoxy ou un groupe acylamino

d) pour l'obtention d'un composé de formule I dans laquelle $R^3$ représente un groupement qui contient le groupe alcanoyloxy en $C_1$ à $C_6$, l'alcanoylation en $C_1$ à $C_6$ sélective d'un composé correspondant de la présente invention dans lequel $R^3$ représente un groupement qui contient un groupe hydroxy

e) pour l'obtention d'un composé de formule I dans laquelle X, $R^1$ et/ou $R^3$ contiennent un groupe $-SO_2$, l'oxydation d'un composé correspondant de la présente invention dans lequel X, $R^1$ et/ou $R^3$ contiennent un groupe -S- ou $-SO_2-$

f) pour l'obtention d'un composé de formule I dans laquelle X, $R^1$ et/ou $R^3$ contiennent un groupe -SO-, l'oxydation d'un composé correspondant de la présente invention dans lequel X, $R^1$ et/ou $R^3$ contiennent un groupe -S- et

g) pour l'obtention d'un composé de formule I dans laquelle $R^1$ et/ou $R^3$ contiennent un groupe alkoxy en $C_1$ à $C_6$, alcényloxy en $C_3$ à $C_6$ ou alcynyloxy en $C_3$ à $C_6$, l'alkylation, l'alcénylation ou l'alcynylation d'un composé correspondant de la présente invention dans lequel $R^1$ et/ou $R^3$ contiennent un groupe hydroxy

h) pour l'obtention d'un composé de formule I dans laquelle $R^1$ et/ou $R^3$ représentent un groupement contenant un groupe acylamino, l'acylation d'un composé correspondant de la présente invention dans lequel $R^1$ et/ou $R^3$ représentent un groupement contenant un groupe amino

après quoi, lorsque le composé de formule I ou un racémate de ce composé est obtenu sous la forme d'une base et que l'on désire un sel, le composé de formule I obtenu est amené à réagir avec un acide pour former le sel.

**2.** Procédé suivant la revendication 1, dans lequel $R^1$ représente un groupe phényle ou naphtyle portant un ou deux substituants choisis entre un halogène, un groupe trifluorométhyle, cyano, nitro, alkyle en $C_1$ à $C_6$ ou alkoxy en $C_1$ à $C_6$ ; ou bien $R^1$ est un noyau hétérocyclique pentagonal ou hexagonal contenant un ou deux hétéroatomes choisis entre oxygène, azote et soufre, le noyau hétérocyclique étant condensé avec un noyau benzénique, et le noyau hétérocyclique et/ou le noyau benzénique étant facultativement substitués par un halogène et/ou un groupe hydroxy, la liaison au reste du composé de formule I s'effectuant par l'intermédiaire du noyau hétérocyclique.

**3.** Procédé suivant la revendication 1 ou 2, dans lequel $R^1$ représente un groupe halogénophényle, dihalogénophényle, trifluorométhylphényle, méthoxyphényle, méthylphényle, nitrophényle, cyanophényle, naphtyle, benzothiényle, benzofurannyle, benzoxazolyle, benzisoxazolyle facultativement substitué par du fluor, benzimidazolyle facultativement substitué par du fluor ou un groupe 2-(1,3-dioxoisoindolinyle).

**4.** Procédé suivant l'une quelconque des revendications précédentes, dans lequel X représente $-CH_2-$.

**5.** Procédé suivant l'une quelconque des revendications précédentes, dans lequel $R^2$ représente le groupe méthyle.

**6.** Procédé suivant l'une quelconque des revendications précédentes, dans lequel $R^3$ représente un groupe alkyle ayant jusqu'à 7 atomes de carbone, facultativement substitué par un radical hydroxy, alkylsulfure en $C_1$ à $C_6$, alkylsulfinyle en $C_1$ à $C_6$ ou alkoxy en $C_1$ à $C_6$ ; ou bien $R^3$ représente un groupe phényle facultativement substitué par un ou deux substituants choisis entre des substituants hydroxy, nitro, alkylsulfure en $C_1$ à $C_6$, alkylsulfinyle en $C_1$ à $C_6$, alkylsulfonyle en $C_1$ à $C_6$, alkoxy en $C_1$ à $C_6$, amino, alkylamino en $C_1$ à $C_6$ ou acylamino en $C_1$ à $C_6$, ou bien $R^3$ représente un groupe de formule $-D-R_6$ dans laquelle D représente une liaison simple ou un groupe $-CH(CH_3)-$ et $R_6$ représente un noyau hétérocyclique pentagonal ou hexagonal contenant un ou deux hétéroatomes choisis entre oxygène, azote ou soufre, ou bien $R^3$ représente un groupe benzyle.

**7.** Procédé suivant l'une quelconque des revendications précédentes, dans lequel $R^3$ représente un groupe isopropyle, isobutyle, sec.-butyle, tertio-butyle, 1-(alkoxy en $C_1$ à $C_4$)éthyle, phényle, hydroxyphényle, 1-méthylthioéthyle, 1-morpholinoéthyle, diméthoxyphényle, hydroxyméthylphényle, aminophényle, acétamidophényle ou méthylaminophényle.

**8.** Procédé suivant la revendication 1 pour l'obtention d'un composé de formule I choisi entre
la (2S)-N-[2-(N-méthyl-3,4-dichlorophénylacétamido)-2-phényléthyl]pyrrolidine.
la (2S)-N-[2-(N-méthyl-4-trifluorométhylphénylacétamido)-2-phényléthyl]pyrrolidine,
la (2S,3R)-N-[2-(N-méthyl-3,4-dichlorophénylacétamido)-3-méthoxybutyl]pyrrolidine, et
la (2S)-N-[2-(N-méthyl-3,4-dichlorophénylacétamido)-2-[phényléthyl]$\Delta^3$-pyrrolidine et leurs sels.

**9.** Procédé suivant l'une quelconque des revendications précédentes, dans lequel le composé de formule (I) ou son racémate est obtenu sous forme d'un sel acceptable du point de vue pharmaceutiques.